# EUROPEAN PATENT APPLICATION

(11) **EP 3 586 782 A1**
(43) Date of publication of application: **01.01.2020**
(21) Application number: 19182796.3
(22) Date of filing: 27.06.2019
(51) Int. Cl.: A61B 34/20, A61B 34/32, A61B 34/37, A61B 90/00, B23Q 17/22, B23Q 17/24, B25J 9/16, A61B 17/00, A61B 34/00

(54) **CONTROLLING A SURGICAL ROBOT TO AVOID ROBOTIC ARM COLLISION**

(30) Priority: 28.06.2018 US 201816021068
(71) Applicant: Globus Medical, Inc., Audubon, PA 19403 (US)
(72) Inventor: CRAWFORD, Neil, CHANDLER, AZ Arizona 85224 (US); JOHNSON, Norbert, NORTH ANDOVER, MA Massachusetts 01845 (US); FORSYTH, Jeffrey, CRANSTON, RI Rhode Island 02921 (US); BRAUCKMANN, Michael, WOBURN, MA Massachusetts 01801 (US)
(74) Representative: Morabito, Sara

(57) **Abstract**

Surgical robotic systems including a surgical robot, a sensor, and a surgical control computer are disclosed. To determine an actual or predicted collision of a robotic arm of the surgical robot with a patient, the sensor is configured to output a proximity signal indicating proximity of the robotic arm to a patient while the robotic arm is adjacent to the patient. A processor of the surgical control computer receives the proximity signal from the sensor and determines when the robotic arm has collided with the patient or is predicted to collide with the patient based on the received proximity signal. In response to determining such an actual or predicted collision, the processor performs a remedial action. By having surgical robotic systems perform remedial action(s) responsive to determining an actual or predicted collision, collisions between the robotic arm and the patient can be reduced and/or eliminated.

## Description

### CROSS REFERENCE TO RELATED APPLICATIONS

This application is a continuation-in-part of U.S. Patent Application Serial No. 15/609,334 which is a continuation-in-part of U.S. Patent Application No. 15/157,444, filed May 18, 2016, which is a continuation-in-part of U.S. Patent Application No. 15/095,883, filed April 11, 2016, which is a continuation-in-part of U.S. Patent Application No. 14/062,707, filed on October 24, 2013, which is a continuation-in-part application of U.S. Patent Application No. 13/924,505, filed on June 21, 2013, which claims priority to provisional application No. 61/662,702 filed on June 21, 2012 and claims priority to provisional application No. 61/800,527 filed on March 15, 2013, all of which are incorporated by reference herein in their entireties for all purposes.

### FIELD OF THE DISCLOSURE

The present disclosure relates to medical devices, and more particularly to surgical robotic systems and related methods and devices.

### BACKGROUND

Advances in technology have recently led to an increase in the use of surgical robotic systems during surgery. Typically, surgical robotic systems include a surgical robot that is manually controlled by a surgeon and/or autonomously controlled by a computer to perform or assist in surgery. In a manually-controlled system, a surgeon may control the surgical robot through either a manipulator and/or a computer control. For example, a surgeon may manually engage a load cell disposed on an end-effector (i.e., a device at the end of a robotic arm of the surgical robot designed to interact with the environment) to cause the end-effector to perform an incision on a patient. In contrast, an autonomously-controlled surgical robotic system may use a computer program to control the surgical robot to perform given movements of a surgery. In some cases, surgical robotic systems may have both manual and autonomous characteristics. By using surgical robotic systems instead of traditional surgical techniques, movements of a surgery may be executed with increased stability, precision, speed, and smoothness. In this regard, surgeries using robotic systems may achieve smaller incisions, reduced tissue trauma, and decreased blood loss, resulting in benefits such as reduced transfusions and scarring, and shorter operation times and healing times.

However, conventional surgical robotic systems may suffer from limited feedback compared to traditional surgical techniques. For example, while a surgeon conducting a traditional surgery by hand may be able to adjust an instrument to a certain angle while simultaneously sensing whether he or she is in contact with the patient, a conventional surgical robotic system may lack the feedback necessary to sense such information. As a result, portions of the surgical robot of such a system may collide with a patient while adjusting to a particular instrument angle or viewpoint. In this regard, conventional surgical robotic systems can cause pain, discomfort, and/or harm to the patient and damage to the surgical robot itself.

### SUMMARY

Aspects disclosed in the detailed description are directed to performing a remedial action in a surgical robotic system in response to determining an actual or predicted collision of a robotic arm. During surgeries, a surgical robot can maneuver surgical instruments at steep angles and in close proximity to a patient. Positioning surgical instruments in such a manner can result in a collision between the surgical robot and the patient, causing injury to the patient and/or damage to the surgical robot. The robotic arm of the surgical robot is at particular risk for such collision due to its proximity to the surgical end-effector.

Thus, in exemplary aspects disclosed herein, surgical robotic systems including a surgical robot, a sensor, and a surgical control computer are provided. To determine an actual or predicted collision of a robotic arm of the surgical robot with a patient, the sensor is configured to output a proximity signal indicating proximity of the robotic arm to a patient while the robotic arm is adjacent to the patient. A processor of the surgical control computer receives the proximity signal from the sensor and determines when the robotic arm has collided with the patient or is predicted to collide with the patient based on the received proximity signal. In response to determining such an actual or predicted collision, the processor performs a remedial action.

In at least one non-limiting embodiment, such as in a manually-controlled surgical robotic system, performing the remedial action includes displaying a collision warning to an operator. In another non-limiting embodiment, such as an autonomously-controlled surgical robotic system, performing the remedial action includes inhibiting movement of the robotic arm in a direction toward where the robotic arm has collided or is predicted to collide with the patient. By having the surgical robotic system perform remedial action(s) responsive to determining an actual or predicted collision, collisions between the robotic arm and the patient can be reduced and/or eliminated without the need for increased surgeon training and/or excessive range of motion restrictions for the robotic arm. In this manner, surgical robotic systems can be used in surgeries to achieve reduced tissue damage, blood loss, and scarring, as well as shorter operation times and healing times, at a reduced cost.

Other methods, surgical robotic systems, and computer program products, according to aspects disclosed herein, will become apparent to one with skill in the art upon review of the following drawings and detailed description. It is intended that all such methods, surgical robotic systems, and computer program products be included within this description, be within the scope of the present inventive subject matter, and be protected by the accompanying claims. Moreover, it is intended that all embodiments disclosed herein can be implemented separately or combined in any way and/or combination.

### DESCRIPTION OF THE DRAWINGS

The accompanying drawings, which are incorporated as a part of this application and included to provide a further understanding of disclosures herein, illustrate exemplary non-limiting embodiments of inventive concepts recited in the claims and elsewhere throughout this application. In this regard, the drawings disclosed herein are directed to the following:
FIG. 1 is an overhead view of an exemplary arrangement for a robotic surgical system, patient, surgeon, and other medical personnel during a surgical procedure;
FIG. 2 illustrates the exemplary robotic surgical system of FIG. 1 including positioning of a surgical robot and a camera relative to the patient according to one exemplary embodiment;
FIG. 3 illustrates a surgical robotic system in accordance with an exemplary embodiment;
FIG. 4 illustrates a portion of a surgical robot in accordance with an exemplary embodiment;
FIG. 5 illustrates a block diagram of the surgical robot illustrated in FIG. 3 in accordance with an exemplary embodiment;
FIG. 6 illustrates a surgical robot in accordance with an exemplary embodiment;
FIGS. 7A-7C illustrate an end-effector in accordance with an exemplary embodiment;
FIG. 8 illustrates a surgical instrument and an end-effector, before and after, inserting the surgical instrument into the guide tube of the end-effector according to one exemplary embodiment;
FIGS. 9A-9C illustrate portions of an end-effector and a robotic arm in accordance with an exemplary embodiment;
FIG. 10 illustrates a dynamic reference array, an imaging array, and other components in accordance with an exemplary embodiment;
FIG. 11 illustrates a method of registration in accordance with an exemplary embodiment;
FIGS. 12A-12B illustrate imaging devices according to exemplary embodiments;
FIG. 13A illustrates a portion of a surgical robot including a robotic arm and an end-effector in accordance with an exemplary embodiment;
FIG. 13B illustrates a close-up view of the end-effector illustrated in FIG. 13A with a plurality of tracking markers rigidly affixed thereon;
FIG. 13C illustrates a tool or instrument with a plurality of tracking markers rigidly affixed thereon according to one exemplary embodiment;
FIG. 14A illustrates an alternative version of an end-effector with moveable tracking markers in a first configuration according to one exemplary embodiment;
FIG. 14B illustrates the end-effector shown in FIG. 14A with the moveable tracking markers in a second configuration;
FIG. 14C shows the template of tracking markers in the first configuration from FIG. 14A;
FIG. 14D shows the template of tracking markers in the second configuration from FIG. 14B;
FIG. 15A illustrates an alternative version of an end-effector having only a single tracking marker affixed thereto according to one exemplary embodiment;
FIG. 15B illustrates the end-effector of FIG. 15A with an instrument disposed through a guide tube;
FIG. 15C illustrates the end-effector of FIG. 15A with the instrument in two different positions, and the resulting logic to determine if the instrument is positioned within the guide tube or outside of the guide tube;
FIG. 15D illustrates the end-effector of FIG. 15A with the instrument in the guide tube at two different frames and its relative distance to the single tracking marker on the guide tube;
FIG. 15E illustrates the end-effector of FIG. 15A relative to a coordinate system;
FIG. 16 is a block diagram of a method for navigating and moving the end-effector of the surgical robot to a desired target trajectory according to one exemplary embodiment;
FIGS. 17A-17B illustrate an instrument for inserting an expandable implant having fixed and moveable tracking markers in contracted and expanded positions, respectively;
FIGS. 18A-18B illustrate an instrument for inserting an articulating implant having fixed and moveable tracking markers in insertion and angled positions, respectively;
FIG. 19A illustrates an embodiment of a surgical robot with interchangeable or alternative end-effectors;
FIG. 19B illustrates an embodiment of a surgical robot with an instrument-style end-effector coupled thereto;
FIG. 20 illustrates a block diagram of a surgical robotic system including a sensor(s) and a surgical control computer connected to a surgical robot to perform remedial action in response to determining an actual or predicted collision of a robotic arm of the surgical robot in accordance with some exemplary embodiments; and
FIGS. 21-24 illustrate flowcharts of operations of the surgical control computer in the surgical robotic system(s) of FIG. 20 according to some exemplary embodiments.

### DETAILED DESCRIPTION

The following discussion is presented to enable a person having ordinary skill in the art to make and use embodiments of the present disclosure. Various modifications to the illustrated embodiments will be readily apparent to a person having ordinary skill in the art. As such, the principles associated with the embodiments disclosed herein, as understood by a person having ordinary skill in the art, will be readily applicable to other embodiments and applications without departing from the embodiments of the present disclosure. Thus, the embodiments disclosed herein are not intended to be limited to only the embodiments shown herein, but are to be accorded the widest scope consistent with the principles and features disclosed herein. In this regard, the exemplary aspects disclosed in the following detailed description are to be read with reference to the figures, in which like elements in different figures may have like reference numerals. The word "exemplary" is used herein to mean "serving as an example, instance, or illustration." Any aspect described herein as "exemplary" should not necessarily be construed as preferred or advantageous over other aspects. The figures, which are not necessarily to scale, depict selected embodiments and are not intended to limit the scope of the embodiments. A person having ordinary skill in the art will recognize that the examples provided herein have many useful alternatives and fall within the scope of the embodiments.

As explained above, during surgeries using surgical robotic systems, it may be desirable to place aspects of a surgical robot, such as a surgical instrument, at steep angles and in close proximity to a patient to perform particular movements of the surgery. However, positioning the surgical robot in such a manner can result in a collision between the surgical robot and the patient, causing injury to the patient and/or damage to the surgical robot. The robotic arm of the surgical robot is at particular risk for such a collision due to its proximity to the surgical end-effector.

Thus, in exemplary aspects disclosed herein, surgical robotic systems including a surgical robot, a sensor, and a surgical control computer are provided. To determine an actual or predicted collision of a robotic arm of the surgical robot with a patient, the sensor is configured to output a proximity signal indicating proximity of the robotic arm to a patient while the robotic arm is adjacent to the patient. A processor of the surgical control computer receives the proximity signal from the sensor and determines when the robotic arm has collided with the patient or is predicted to collide with the patient based on the received proximity signal. In response to determining such an actual or predicted collision, the processor performs a remedial action. In at least one non-limiting embodiment, such as in a manually-controlled surgical robotic system, performing the remedial action includes displaying a collision warning to an operator. In another non-limiting embodiment, such as an autonomously-controlled surgical robotic system, performing the remedial action includes inhibiting movement of the robotic arm in a direction toward where the robotic arm has collided or is predicted to collide with the patient. By having the surgical robotic system perform remedial action(s) responsive to determining an actual or predicted collision, collisions between the robotic arm and the patient can be reduced and/or eliminated without the need for increased surgeon training and/or excessive range of motion restrictions for the robotic arm. In this manner, surgical robotic systems can be used in surgeries to achieve reduced tissue damage, blood loss, and scarring, as well as shorter operation times and healing times, at a reduced cost.

Although various embodiments are described in the context of performing remedial action in a surgical robotic system in response to determining an actual or predicted collision of a robotic arm, this disclosure is not limited thereto. An example surgical robotic system is initially described below in detail followed by a description of various configurations and operations associated with performing remedial action in a surgical robotic system in response to determining an actual or predicted collision of a robotic arm in accordance with embodiments of the present disclosure.

### surgical robotic system

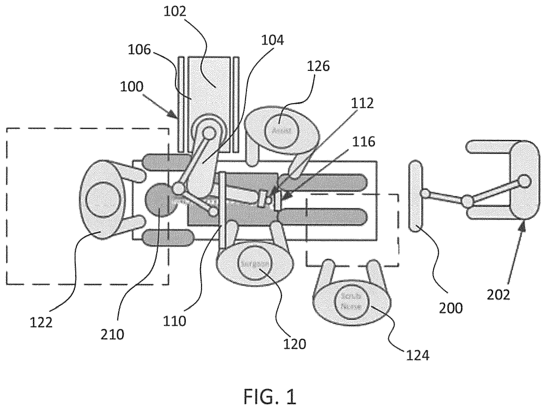

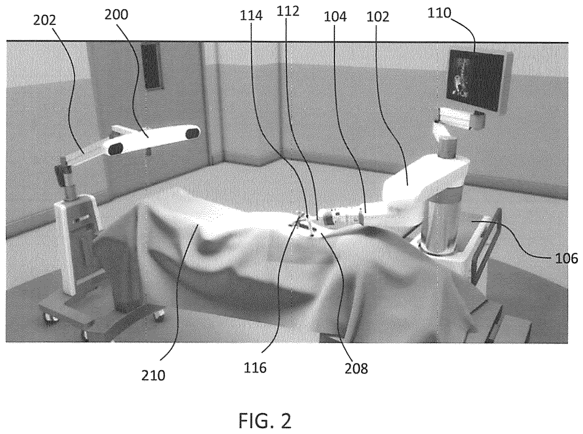

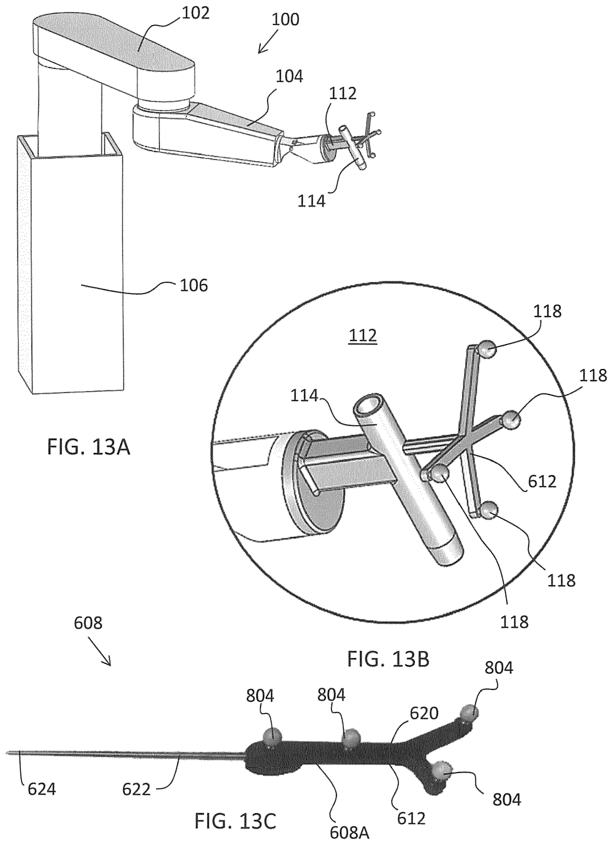

Turning now to the drawings, FIGS. 1 and 2 illustrate a surgical robotic system 100 in accordance with an exemplary embodiment. Surgical robotic system 100 may include, for example, a surgical robot 102, one or more robotic arms 104, a base 106, a display 110, and an end-effector 112 including, for example, a guide tube 114 (illustrated in FIG. 2). The surgical robotic system 100 may include a patient tracking device 116 also including one or more tracking markers 118, wherein the patient tracking device 116 is secured directly to the patient 210 (e.g., to a bone of the patient 210). The surgical robotic system 100 may also use a camera 200, for example, positioned on a camera stand 202. The camera stand 202 can have any suitable configuration to move, orient, and support the camera 200 in a desired position. The camera 200 may include any suitable camera or cameras, such as one or more infrared cameras (e.g., bifocal or stereophotogrammetrical cameras), able to identify, for example, active and passive tracking markers 118 (shown as part of the patient tracking device 116 illustrated in FIG. 2 and shown in an enlarged view in FIGS. 13A-13B) in a given measurement volume viewable from the perspective of the camera 200. The camera 200 may scan the given measurement volume and detect the light that comes from the markers 118 in order to identify and determine the position of the markers 118 in three-dimensions. For example, active markers 118 may include infrared-emitting markers that are activated by an electrical signal (e.g., infrared (IR) light emitting diodes (LEDs)), and/or passive markers 118 may include retro-reflective markers that reflect IR light (e.g., they reflect incoming IR radiation into the direction of the incoming light), for example, emitted by illuminators on the camera 200 or other suitable device.

FIGS. 1 and 2 illustrate one exemplary configuration of the surgical robotic system 100 in an operating room environment. As illustrated in FIGS. 1 and 2, the surgical robot 102 may be positioned near or adjacent to the patient 210. Although depicted near the head of the patient 210, it will be appreciated that the surgical robot 102 can be positioned at any suitable location near the patient 210 depending on the area of the patient 210 undergoing the operation. The camera 200 may be separated from the surgical robotic system 100 and positioned at the foot of the patient 210. This location allows the camera 200 to have a direct visual line of sight to the surgical field 208. Again, it is contemplated that the camera 200 may be located at any suitable position having line of sight to the surgical field 208. In the configuration shown, the surgeon 120 may be positioned across from the surgical robot 102, but still able to manipulate the end-effector 112 and the display 110. A surgical assistant 126 may be positioned across from the surgeon 120 with access to both the end-effector 112 and the display 110. If desired, the locations of the surgeon 120 and the assistant 126 may be reversed. The traditional areas for the anesthesiologist 122 and the nurse or scrub tech 124 may remain unimpeded by the locations of the robot 102 and camera 200.

With respect to the other components of the surgical robot 102, the display 110 can be attached to the surgical robot 102. In other exemplary embodiments, the display 110 can be detached from the surgical robot 102, either within a surgical room with the surgical robot 102, or in a remote location. The end-effector 112 may be coupled to the robotic arm 104 and controlled by at least one motor. In exemplary embodiments, the end-effector 112 can include a guide tube 114, which is able to receive and/or orient a surgical instrument 608 (described further below) used to perform surgery on the patient 210. As used herein, the term "end-effector" is used interchangeably with the terms "end-effectuator" and "effectuator element." Although generally shown with the guide tube 114, it will be appreciated that the end-effector 112 may be replaced with any suitable instrumentation suitable for use in surgery. In some embodiments, the end-effector 112 can comprise any known structure for effecting the movement of the surgical instrument 608 in a desired manner.

The surgical robot 102 is able to control the translation and orientation of the end-effector 112. The surgical robot 102 is able to move the end-effector 112 along x-, y-, and z-axes, for example. The end-effector 112 can be configured for selective rotation about one or more of the x-, y-, and z-axis , and a Z Frame axis (such that one or more of the Euler Angles (e.g., roll, pitch, and/or yaw) associated with the end-effector 112 can be selectively controlled). In some exemplary embodiments, selective control of the translation and orientation of the end-effector 112 can permit performance of medical procedures with significantly improved accuracy compared to conventional robots that use, for example, a robotic arm having six degrees of freedom and comprising only rotational axes. For example, the surgical robotic system 100 may be used to operate on the patient 210, and the robotic arm 104 can be positioned above the body of the patient 210, with the end-effector 112 selectively angled relative to the z-axis toward the body of the patient 210.

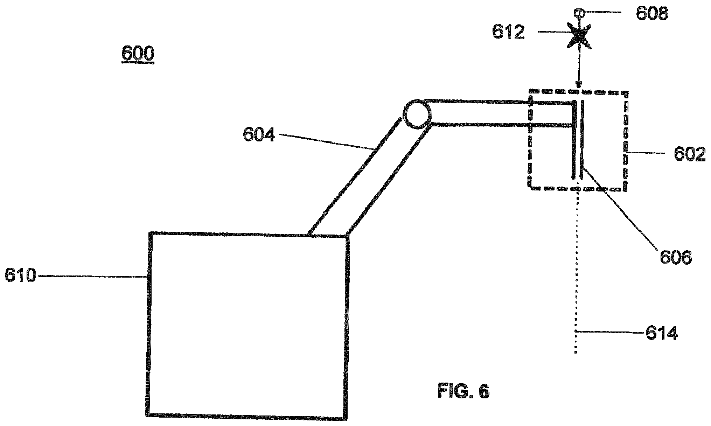

In some exemplary embodiments, the position of the surgical instrument (e.g., the surgical instrument 608 illustrated in FIG. 6) can be dynamically updated so that the surgical robot 102 can be aware of the location of the surgical instrument 608 at all times during the procedure. Consequently, in some exemplary embodiments, the surgical robot 102 can move the surgical instrument 608 to the desired position quickly without any further assistance from a physician (unless the physician so desires). In some further embodiments, the surgical robot 102 can be configured to correct the path of the surgical instrument 608 if the surgical instrument 608 strays from the selected, preplanned trajectory. In some exemplary embodiments, the surgical robot 102 can be configured to permit stoppage, modification, and/or manual control of the movement of the end-effector 112 and/or the surgical instrument 608. Thus, in use, in exemplary embodiments, a physician or other user can operate the surgical robotic system 100, and has the option to stop, modify, or manually control the autonomous movement of the end-effector 112 and/or the surgical instrument 608. Further details of the surgical robotic system 100 including the control and movement of a surgical instrument 608 by a surgical robot 102 can be found in U.S. patent application Ser. No. 13/924,505, which is incorporated herein by reference in its entirety.

The robotic surgical system 100 can comprise one or more tracking markers 118 configured to track the movement of the robotic arm 104, the end-effector 112, the patient 210, and/or the surgical instrument 608 in three dimensions. In exemplary embodiments, a plurality of tracking markers 118 can be mounted (or otherwise secured) to an outer surface of the surgical robot 102, such as, for example, on the base 106 of the surgical robot 102, on the robotic arm 104, and/or on the end-effector 112. In exemplary embodiments, at least one tracking marker 118 of the plurality of tracking markers 118 can be mounted or otherwise secured to the end-effector 112. One or more tracking markers 118 can further be mounted (or otherwise secured) to the patient 210. In exemplary embodiments, the plurality of tracking markers 118 can be positioned on the patient 210 spaced apart from the surgical field 208 to reduce the likelihood of being obscured by the surgeon, surgical tools, or other parts of the robot 102. Further, one or more tracking markers 118 can be further mounted (or otherwise secured) to the surgical tools 608 (e.g., a screw driver, dilator, implant inserter, or the like). Thus, the tracking markers 118 enable each of the marked objects (e.g., the end-effector 112, the patient 210, and the surgical tools 608) to be tracked by the robot 102. In exemplary embodiments, the surgical robotic system 100 can use tracking information collected from each of the marked objects to calculate the orientation and location, for example, of the end-effector 112, the surgical instrument 608 (e.g., positioned in the tube 114 of the end-effector 112), and the relative position of the patient 210.

The markers 118 may include radiopaque or optical markers. The markers 118 may be suitably shaped include spherical, spheroid, cylindrical, cube, cuboid, or the like. In exemplary embodiments, one or more of markers 118 may be optical markers. In some embodiments, the positioning of one or more tracking markers 118 on end-effector 112 can maximize the accuracy of the positional measurements by serving to check or verify the position of end-effector 112. Further details of the surgical robotic system 100 including the control, movement and tracking of surgical robot 102 and of a surgical instrument 608 can be found in U.S. patent publication No. 2016/0242849, which is incorporated herein by reference in its entirety.

Exemplary embodiments include one or more markers 118 coupled to the surgical instrument 608. In exemplary embodiments, these markers 118, for example, coupled to the patient 210 and surgical instruments 608, as well as markers 118 coupled to the end-effector 112 of the robot 102 can comprise conventional infrared light-emitting diodes (LEDs) or an Optotrak® diode capable of being tracked using a commercially available infrared optical tracking system such as Optotrak®. Optotrak® is a registered trademark of Northern Digital Inc., Waterloo, Ontario, Canada. In other embodiments, markers 118 can comprise conventional reflective spheres capable of being tracked using a commercially available optical tracking system such as Polaris Spectra. Polaris Spectra is also a registered trademark of Northern Digital, Inc. In an exemplary embodiment, the markers 118 coupled to the end-effector 112 are active markers which comprise infrared light-emitting diodes which may be turned on and off, and the markers 118 coupled to the patient 210 and the surgical instruments 608 comprise passive reflective spheres.

In exemplary embodiments, light emitted from and/or reflected by markers 118 can be detected by camera 200 and can be used to monitor the location and movement of the marked objects. In alternative embodiments, markers 118 can comprise a radio-frequency and/or electromagnetic reflector or transceiver and the camera 200 can include or be replaced by a radio-frequency and/or electromagnetic transceiver.

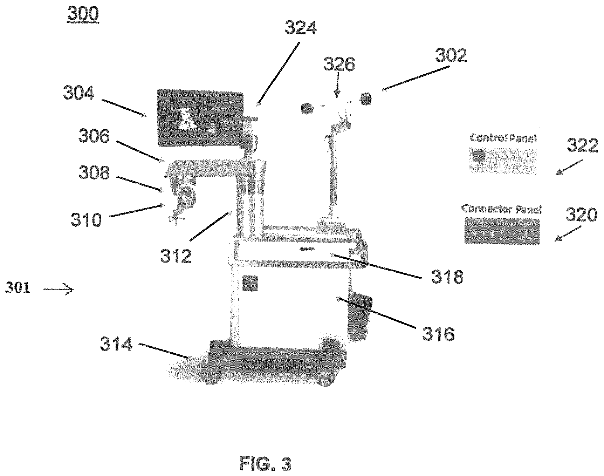

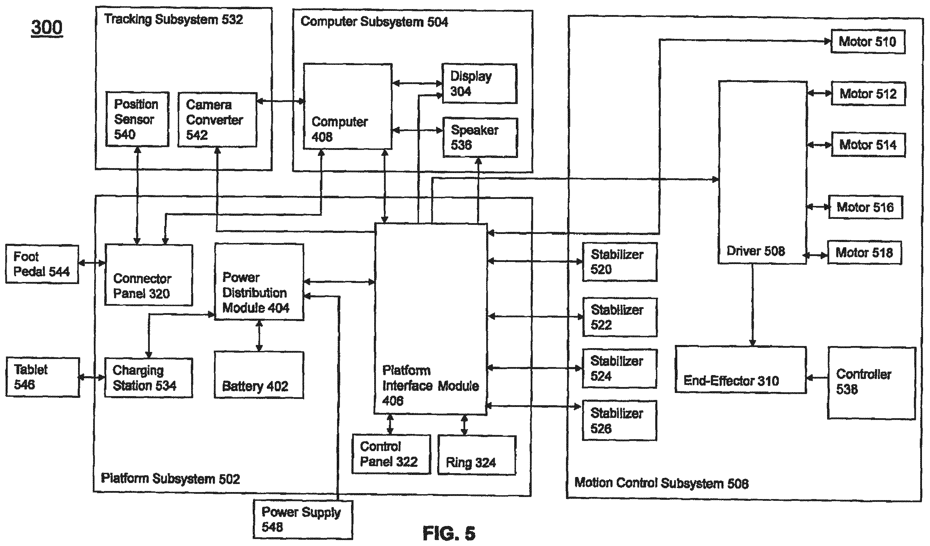

Similar to the surgical robotic system 100, FIG. 3 illustrates a surgical robotic system 300 and camera stand 302, in a docked configuration, consistent with an exemplary embodiment of the present disclosure. Surgical robotic system 300 may comprise a robot 301 including a display 304, upper arm 306, lower arm 308, end-effector 310, vertical column 312, casters 314, cabinet 316, tablet drawer 318, connector panel 320, control panel 322, and ring of information 324. Camera stand 302 may comprise camera 326. These components are described in greater with respect to FIG. 5. FIG. 3 illustrates the surgical robotic system 300 in a docked configuration where the camera stand 302 is nested with the robot 301, for example, when not in use. It will be appreciated by those skilled in the art that the camera 326 and robot 301 may be separated from one another and positioned at any appropriate location during the surgical procedure, for example, as shown in FIGS. 1 and 2.

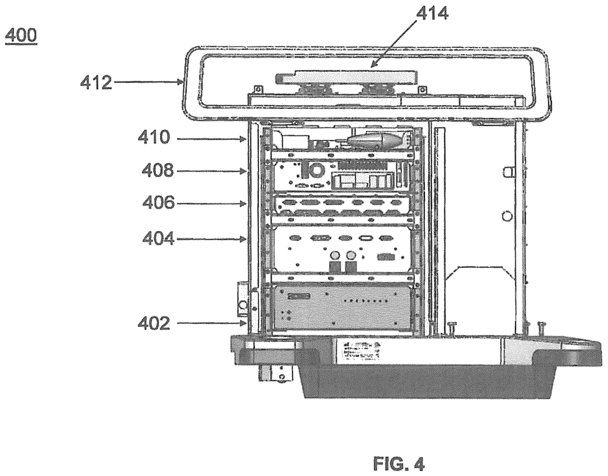

FIG. 4 illustrates a base 400 consistent with an exemplary embodiment of the present disclosure. Base 400 may be a portion of surgical robotic system 300 and comprise cabinet 316. Cabinet 316 may house certain components of surgical robotic system 300 including but not limited to a battery 402, a power distribution module 404, a platform interface board module 406, a computer 408, a handle 412, and a tablet drawer 414. The connections and relationship between these components is described in greater detail with respect to FIG. 5.

FIG. 5 illustrates a block diagram of certain components of an exemplary embodiment of surgical robotic system 300. Surgical robotic system 300 may comprise platform subsystem 502, computer subsystem 504, motion control subsystem 506, and tracking subsystem 532. Platform subsystem 502 may further comprise battery 402, power distribution module 404, platform interface board module 406, and tablet charging station 534. Computer subsystem 504 may further comprise computer 408, display 304, and speaker 536. Motion control subsystem 506 may further comprise driver circuit 508, motors 510, 512, 514, 516, 518, stabilizers 520, 522, 524, 526, end-effector 310, and controller 538. Tracking subsystem 532 may further comprise position sensor 540 and camera converter 542. System 300 may also comprise a foot pedal 544 and tablet 546.

Input power is supplied to system 300 via a power source 548 which may be provided to power distribution module 404. Power distribution module 404 receives input power and is configured to generate different power supply voltages that are provided to other modules, components, and subsystems of system 300. Power distribution module 404 may be configured to provide different voltage supplies to platform interface module 406, which may be provided to other components such as computer 408, display 304, speaker 536, driver 508 to, for example, power motors 512, 514, 516, 518 and end-effector 310, motor 510, ring 324, camera converter 542, and other components for system 300 for example, fans for cooling the electrical components within cabinet 316.

Power distribution module 404 may also provide power to other components such as tablet charging station 534 that may be located within tablet drawer 318. Tablet charging station 534 may be in wireless or wired communication with tablet 546 for charging table 546. Tablet 546 may be used by a surgeon consistent with the present disclosure and described herein.

Power distribution module 404 may also be connected to battery 402, which serves as temporary power source in the event that power distribution module 404 does not receive power from input power 548. At other times, power distribution module 404 may serve to charge battery 402 if necessary.

Other components of platform subsystem 502 may also include connector panel 320, control panel 322, and ring 324. Connector panel 320 may serve to connect different devices and components to system 300 and/or associated components and modules. Connector panel 320 may contain one or more ports that receive lines or connections from different components. For example, connector panel 320 may have a ground terminal port that may ground system 300 to other equipment, a port to connect foot pedal 544 to system 300, a port to connect to tracking subsystem 532, which may comprise position sensor 540, camera converter 542, and cameras 326 associated with camera stand 302. Connector panel 320 may also include other ports to allow USB, Ethernet, HDMI communications to other components, such as computer 408.

Control panel 322 may provide various buttons or indicators that control operation of system 300 and/or provide information regarding system 300. For example, control panel 322 may include buttons to power on or off system 300, lift or lower vertical column 312, and lift or lower stabilizers 520-526 that may be designed to engage casters 314 to lock system 300 from physically moving. Other buttons may stop system 300 in the event of an emergency, which may remove all motor power and apply mechanical brakes to stop all motion from occurring. Control panel 322 may also have indicators notifying the user of certain system conditions such as a line power indicator or status of charge for battery 402.

Ring 324 may be a visual indicator to notify the user of system 300 of different modes that system 300 is operating under and certain warnings to the user.

Computer subsystem 504 includes computer 408, display 304, and speaker 536. Computer 504 includes an operating system and software to operate system 300. Computer 504 may receive and process information from other components (for example, tracking subsystem 532, platform subsystem 502, and/or motion control subsystem 506) in order to display information to the user. Further, computer subsystem 504 may also include speaker 536 to provide audio to the user.

Tracking subsystem 532 may include position sensor 504 and converter 542. Tracking subsystem 532 may correspond to camera stand 302 including camera 326 as described with respect to FIG. 3. Position sensor 504 may be camera 326. Tracking subsystem may track the location of certain markers that are located on the different components of system 300 and/or instruments used by a user during a surgical procedure. This tracking may be conducted in a manner consistent with the present disclosure including the use of infrared technology that tracks the location of active or passive elements, such as LEDs or reflective markers, respectively. The location, orientation, and position of structures having these types of markers may be provided to computer 408 which may be shown to a user on display 304. For example, a surgical instrument 608 having these types of markers and tracked in this manner (which may be referred to as a navigational space) may be shown to a user in relation to a three dimensional image of a patient's anatomical structure.

Motion control subsystem 506 may be configured to physically move vertical column 312, upper arm 306, lower arm 308, or rotate end-effector 310. The physical movement may be conducted through the use of one or more motors 510-518. For example, motor 510 may be configured to vertically lift or lower vertical column 312. Motor 512 may be configured to laterally move upper arm 308 around a point of engagement with vertical column 312 as shown in FIG. 3. Motor 514 may be configured to laterally move lower arm 308 around a point of engagement with upper arm 308 as shown in FIG. 3. Motors 516 and 518 may be configured to move end-effector 310 in a manner such that one may control the roll and one may control the tilt, thereby providing multiple angles that end-effector 310 may be moved. These movements may be achieved by controller 538 which may control these movements through load cells disposed on end-effector 310 and activated by a user engaging these load cells to move system 300 in a desired manner.

Moreover, system 300 may provide for automatic movement of vertical column 312, upper arm 306, and lower arm 308 through a user indicating on display 304 (which may be a touchscreen input device) the location of a surgical instrument or component on a three dimensional image of the patient's anatomy on display 304. The user may initiate this automatic movement by stepping on foot pedal 544 or some other input means.

FIG. 6 illustrates a surgical robotic system 600 consistent with an exemplary embodiment. Surgical robotic system 600 may comprise end-effector 602, robotic arm 604, guide tube 606, instrument 608, and robot base 610. Instrument tool 608 may be attached to a tracking array 612 including one or more tracking markers (such as markers 118) and have an associated trajectory 614. Trajectory 614 may represent a path of movement that instrument tool 608 is configured to travel once it is positioned through or secured in guide tube 606, for example, a path of insertion of instrument tool 608 into a patient. In an exemplary operation, robot base 610 may be configured to be in electronic communication with robotic arm 604 and end-effector 602 so that surgical robotic system 600 may assist a user (for example, a surgeon) in operating on the patient 210. Surgical robotic system 600 may be consistent with the previously-described surgical robotic system(s) 100 and 300.

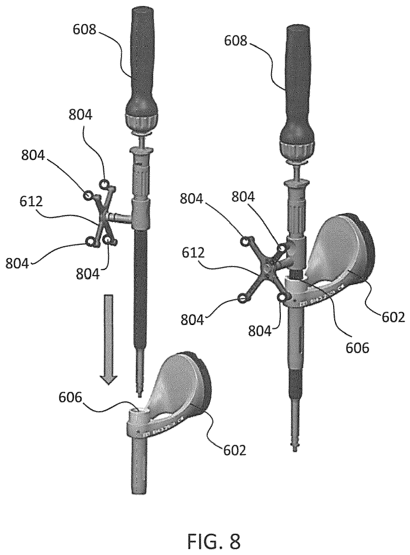

A tracking array 612 may be mounted on instrument 608 to monitor the location and orientation of instrument tool 608. The tracking array 612 may be attached to an instrument 608 and may comprise tracking markers 804. As best seen in FIG. 8, tracking markers 804 may be, for example, light emitting diodes and/or other types of reflective markers (e.g., markers 118 as described elsewhere herein). The tracking devices may be one or more line of sight devices associated with the surgical robotic system. As an example, the tracking devices may be one or more cameras 200, 326 associated with the surgical robotic system 100, 300 and may also track tracking array 612 for a defined domain or relative orientations of the instrument 608 in relation to the robotic arm 604, the robot base 610, end-effector 602, and/or the patient 210. The tracking devices may be consistent with those structures described in connection with camera stand 302 and tracking subsystem 532.

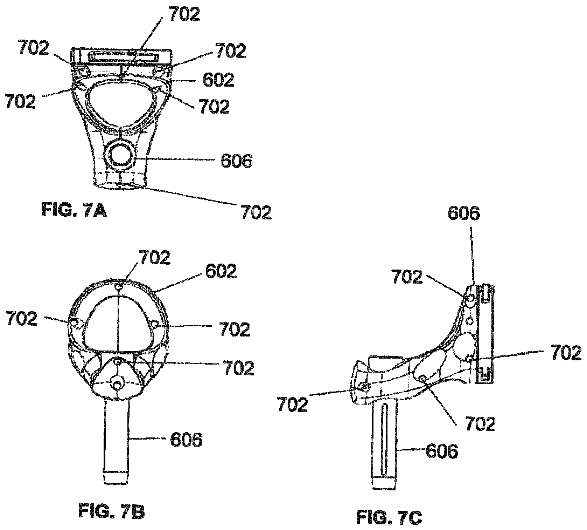

FIGS. 7A, 7B, and 7C illustrate a top view, front view, and side view, respectively, of end-effector 602 consistent with an exemplary embodiment. End-effector 602 may comprise one or more tracking markers 702. Tracking markers 702 may be light emitting diodes or other types of active and passive markers, such as tracking markers 118 that have been previously described. In an exemplary embodiment, the tracking markers 702 are active infrared-emitting markers that are activated by an electrical signal (e.g., infrared light emitting diodes (LEDs)). Thus, tracking markers 702 may be activated such that the infrared markers 702 are visible to the camera 200, 326 or may be deactivated such that the infrared markers 702 are not visible to the camera 200, 326. Thus, when the markers 702 are active, the end-effector 602 may be controlled by the surgical robotic system(s) 100, 300, 600, and when the markers 702 are deactivated, the end-effector 602 may be locked in position and unable to be moved by the surgical robotic system(s) 100, 300, 600.

Markers 702 may be disposed on or within end-effector 602 in a manner such that the markers 702 are visible by one or more cameras 200, 326 or other tracking devices associated with the surgical robotic system 100, 300, 600. The camera 200, 326 or other tracking devices may track end-effector 602 as it moves to different positions and viewing angles by following the movement of tracking markers 702. The location of markers 702 and/or end-effector 602 may be shown on a display 110, 304 associated with the surgical robotic system 100, 300, 600, for example, display 110 as shown in FIG. 2 and/or display 304 shown in FIG. 3. This display 110, 304 may allow a user to ensure that end-effector 602 is in a desirable position in relation to robotic arm 604, robot base 610, the patient 210, and/or the user.

For example, as shown in FIG. 7A, markers 702 may be placed around the surface of end-effector 602 so that a tracking device placed away from the surgical field 208 and facing toward the robot 102, 301 and the camera 200, 326 is able to view at least 3 of the markers 702 through a range of common orientations of the end-effector 602 relative to the tracking device. For example, distribution of markers 702 in this way allows end-effector 602 to be monitored by the tracking devices when end-effector 602 is translated and rotated in the surgical field 208.

In addition, in exemplary embodiments, end-effector 602 may be equipped with infrared (IR) receivers that can detect when an external camera 200, 326 is getting ready to read markers 702. Upon this detection, end-effector 602 may then illuminate markers 702. The detection by the IR receivers that the external camera 200, 326 is ready to read markers 702 may signal the need to synchronize a duty cycle of markers 702, which may be light emitting diodes, to an external camera 200, 326. This may also allow for lower power consumption by the robotic system as a whole, whereby markers 702 would only be illuminated at the appropriate time instead of being illuminated continuously. Further, in exemplary embodiments, markers 702 may be powered off to prevent interference with other navigation tools, such as different types of surgical instruments 608.

FIG. 8 depicts one type of surgical instrument 608 including a tracking array 612 and tracking markers 804. Tracking markers 804 may be of any type described herein including but not limited to light emitting diodes or reflective spheres. Markers 804 are monitored by tracking devices associated with the surgical robotic system 100, 300, 600 and may be one or more of the line of sight cameras 200, 326. The cameras 200, 326 may track the location of instrument 608 based on the position and orientation of tracking array 612 and markers 804. A user, such as a surgeon 120, may orient instrument 608 in a manner so that tracking array 612 and markers 804 are sufficiently recognized by the tracking device or camera 200, 326 to display instrument 608 and markers 804 on, for example, display 110 of the exemplary surgical robotic system.

The manner in which a surgeon 120 may place instrument 608 into guide tube 606 of the end-effector 602 and adjust the instrument 608 is evident in FIG. 8. The hollow tube or guide tube 114, 606 of the end-effector 112, 310, 602 is sized and configured to receive at least a portion of the surgical instrument 608. The guide tube 114, 606 is configured to be oriented by the robotic arm 104 such that insertion and trajectory for the surgical instrument 608 is able to reach a desired anatomical target within or upon the body of the patient 210. The surgical instrument 608 may include at least a portion of a generally cylindrical instrument. Although a screw driver is exemplified as the surgical tool 608, it will be appreciated that any suitable surgical tool 608 may be positioned by the end-effector 602. By way of example, the surgical instrument 608 may include one or more of a guide wire, cannula, a retractor, a drill, a reamer, a screw driver, an insertion tool, a removal tool, or the like. Although the hollow tube 114, 606 is generally shown as having a cylindrical configuration, it will be appreciated by those of skill in the art that the guide tube 114, 606 may have any suitable shape, size and configuration desired to accommodate the surgical instrument 608 and access the surgical site.

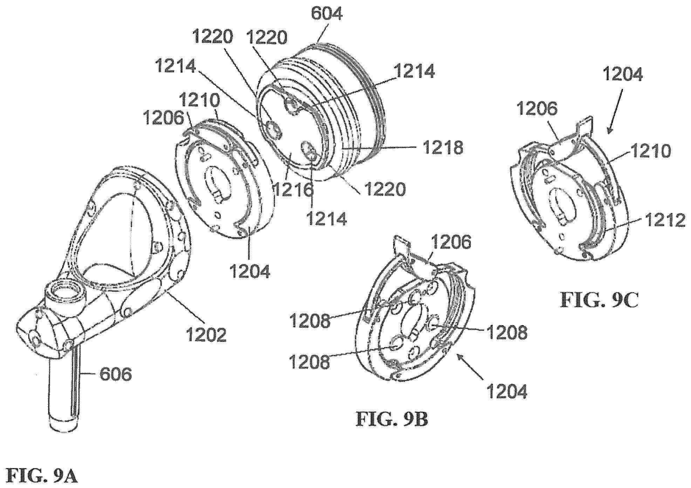

FIGS. 9A-9C illustrate end-effector 602 and a portion of robotic arm 604 consistent with an exemplary embodiment. End-effector 602 may further comprise body 1202 and clamp 1204. Clamp 1204 may comprise handle 1206, balls 1208, spring 1210, and lip 1212. Robotic arm 604 may further comprise depressions 1214, mounting plate 1216, lip 1218, and magnets 1220.

End-effector 602 may mechanically interface and/or engage with the surgical robotic system and robotic arm 604 through one or more couplings. For example, end-effector 602 may engage with robotic arm 604 through a locating coupling and/or a reinforcing coupling. Through these couplings, end-effector 602 may fasten with robotic arm 604 outside a flexible and sterile barrier. In an exemplary embodiment, the locating coupling may be a magnetically kinematic mount and the reinforcing coupling may be a five bar over center clamping linkage.

With respect to the locating coupling, robotic arm 604 may comprise mounting plate 1216, which may be non-magnetic material, one or more depressions 1214, lip 1218, and magnets 1220. Magnet 1220 is mounted below each of depressions 1214. Portions of clamp 1204 may comprise magnetic material and be attracted by one or more magnets 1220. Through the magnetic attraction of clamp 1204 and robotic arm 604, balls 1208 become seated into respective depressions 1214. For example, balls 1208 as shown in FIG. 9B would be seated in depressions 1214 as shown in FIG. 9A. This seating may be considered a magnetically-assisted kinematic coupling. Magnets 1220 may be configured to be strong enough to support the entire weight of end-effector 602 regardless of the orientation of end-effector 602. The locating coupling may be any style of kinematic mount that uniquely restrains six degrees of freedom.

With respect to the reinforcing coupling, portions of clamp 1204 may be configured to be a fixed ground link and as such clamp 1204 may serve as a five bar linkage. Closing clamp handle 1206 may fasten end-effector 602 to robotic arm 604 as lip 1212 and lip 1218 engage clamp 1204 in a manner to secure end-effector 602 and robotic arm 604. When clamp handle 1206 is closed, spring 1210 may be stretched or stressed while clamp 1204 is in a locked position. The locked position may be a position that provides for linkage past center. Because of a closed position that is past center, the linkage will not open absent a force applied to clamp handle 1206 to release clamp 1204. Thus, in a locked position end-effector 602 may be robustly secured to robotic arm 604.

Spring 1210 may be a curved beam in tension. Spring 1210 may be comprised of a material that exhibits high stiffness and high yield strain such as virgin PEEK (poly-ether-etherketone). The linkage between end-effector 602 and robotic arm 604 may provide for a sterile barrier between end-effector 602 and robotic arm 604 without impeding fastening of the two couplings.

The reinforcing coupling may be a linkage with multiple spring members. The reinforcing coupling may latch with a cam or friction based mechanism. The reinforcing coupling may also be a sufficiently powerful electromagnet that will support fastening end-effector 102 to robotic arm 604. The reinforcing coupling may be a multi-piece collar completely separate from either end-effector 602 and/or robotic arm 604 that slips over an interface between end-effector 602 and robotic arm 604 and tightens with a screw mechanism, an over center linkage, or a cam mechanism.

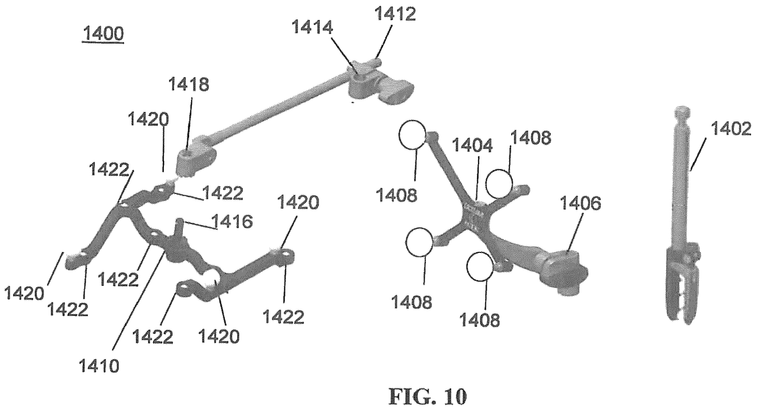

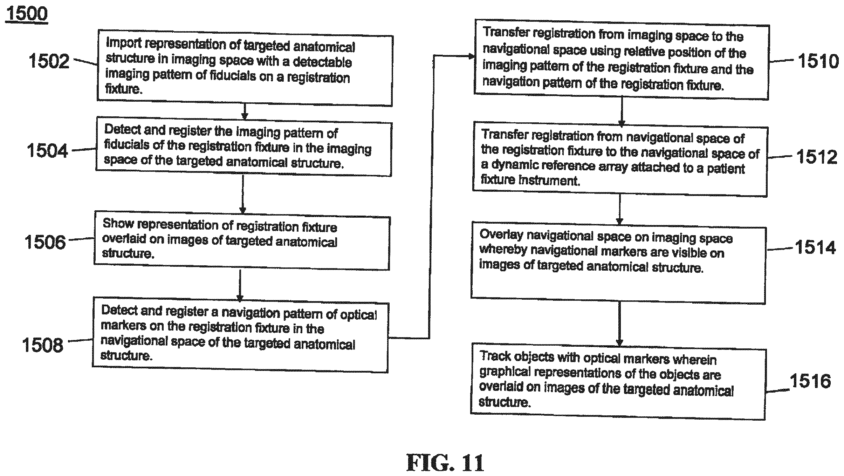

Referring to FIGS. 10 and 11, prior to or during a surgical procedure, certain registration procedures may be conducted to track objects and a target anatomical structure of the patient 210 both in a navigation space and an image space. To conduct such registration, a registration system 1400 may be used as illustrated in FIG. 10.

To track the position of the patient 210, a patient tracking device 116 may include a patient fixation instrument 1402 to be secured to a rigid anatomical structure of the patient 210 and a dynamic reference base (DRB) 1404 may be securely attached to the patient fixation instrument 1402. For example, patient fixation instrument 1402 may be inserted into opening 1406 of dynamic reference base 1404. Dynamic reference base 1404 may contain markers 1408 that are visible to tracking devices, such as tracking subsystem 532. These markers 1408 may be optical markers or reflective spheres, such as tracking markers 118, as previously discussed herein.

Patient fixation instrument 1402 is attached to a rigid anatomy of the patient 210 and may remain attached throughout the surgical procedure. In an exemplary embodiment, patient fixation instrument 1402 is attached to a rigid area of the patient 210, for example, a bone that is located away from the targeted anatomical structure subject to the surgical procedure. In order to track the targeted anatomical structure, dynamic reference base 1404 is associated with the targeted anatomical structure through the use of a registration fixture that is temporarily placed on or near the targeted anatomical structure in order to register the dynamic reference base 1404 with the location of the targeted anatomical structure.

A registration fixture 1410 is attached to patient fixation instrument 1402 through the use of a pivot arm 1412. Pivot arm 1412 is attached to patient fixation instrument 1402 by inserting patient fixation instrument 1402 through an opening 1414 of registration fixture 1410. Pivot arm 1412 is attached to registration fixture 1410 by, for example, inserting a knob 1416 through an opening 1418 of pivot arm 1412.

Using pivot arm 1412, registration fixture 1410 may be placed over the targeted anatomical structure and its location may be determined in an image space and navigation space using tracking markers 1420 and/or fiducials 1422 on registration fixture 1410. Registration fixture 1410 may contain a collection of markers 1420 that are visible in a navigational space (for example, markers 1420 may be detectable by tracking subsystem 532). Tracking markers 1420 may be optical markers visible in infrared light as previously described herein. Registration fixture 1410 may also contain a collection of fiducials 1422, for example, such as bearing balls, that are visible in an imaging space (for example, a three dimension CT image). As described in greater detail with respect to FIG. 11, using registration fixture 1410, the targeted anatomical structure may be associated with dynamic reference base 1404 thereby allowing depictions of objects in the navigational space to be overlaid on images of the anatomical structure. Dynamic reference base 1404, located at a position away from the targeted anatomical structure, may become a reference point thereby allowing removal of registration fixture 1410 and/or pivot arm 1412 from the surgical area.

FIG. 11 provides an exemplary method 1500 for registration consistent with the present disclosure. Method 1500 begins at step 1502 wherein a graphical representation (or image(s)) of the targeted anatomical structure may be imported into system 100, 300 600, for example computer 408. The graphical representation may be three dimensional CT or a fluoroscope scan of the targeted anatomical structure of the patient 210 which includes registration fixture 1410 and a detectable imaging pattern of fiducials 1420.

At step 1504, an imaging pattern of fiducials 1420 is detected and registered in the imaging space and stored in computer 408. Optionally, at this time at step 1506, a graphical representation of the registration fixture 1410 may be overlaid on the images of the targeted anatomical structure.

At step 1508, a navigational pattern of registration fixture 1410 is detected and registered by recognizing markers 1420. Markers 1420 may be optical markers that are recognized in the navigation space through infrared light by tracking subsystem 532 via position sensor 540. Thus, the location, orientation, and other information of the targeted anatomical structure is registered in the navigation space. Therefore, registration fixture 1410 may be recognized in both the image space through the use of fiducials 1422 and the navigation space through the use of markers 1420. At step 1510, the registration of registration fixture 1410 in the image space is transferred to the navigation space. This transferal is done, for example, by using the relative position of the imaging pattern of fiducials 1422 compared to the position of the navigation pattern of markers 1420.

At step 1512, registration of the navigation space of registration fixture 1410 (having been registered with the image space) is further transferred to the navigation space of dynamic registration array 1404 attached to patient fixture instrument 1402. Thus, registration fixture 1410 may be removed and dynamic reference base 1404 may be used to track the targeted anatomical structure in both the navigation and image space because the navigation space is associated with the image space.

At steps 1514 and 1516, the navigation space may be overlaid on the image space and objects with markers visible in the navigation space (for example, surgical instruments 608 with optical markers 804). The objects may be tracked through graphical representations of the surgical instrument 608 on the images of the targeted anatomical structure.

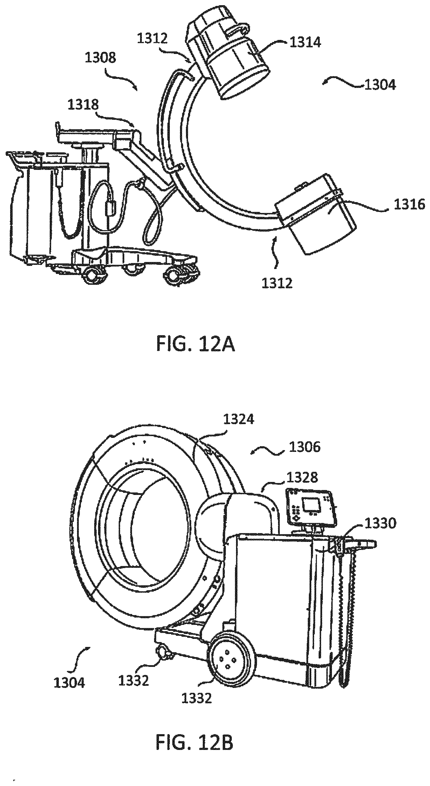

FIGS. 12A-12B illustrate imaging devices 1304 that may be used in conjunction with robot systems 100, 300, 600 to acquire pre-operative, intra-operative, post-operative, and/or real-time image data of patient 210. Any appropriate subject matter may be imaged for any appropriate procedure using the imaging system 1304. The imaging system 1304 may be any imaging device such as imaging device 1306 and/or a C-arm 1308 device. It may be desirable to take x-rays of patient 210 from a number of different positions, without the need for frequent manual repositioning of patient 210 which may be required in an x-ray system. As illustrated in FIG. 12A, the imaging system 1304 may be in the form of a C-arm 1308 that includes an elongated C-shaped member terminating in opposing distal ends 1312 of the "C" shape. C-shaped member 1130 may further comprise an x-ray source 1314 and an image receptor 1316. The space within C-arm 1308 of the arm may provide room for the physician to attend to the patient substantially free of interference from x-ray support structure 1318. As illustrated in FIG. 12B, the imaging system may include imaging device 1306 having a gantry housing 1324 attached to a support structure imaging device support structure 1328, such as a wheeled mobile cart 1330 with wheels 1332, which may enclose an image capturing portion, not illustrated. The image capturing portion may include an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of patient 210 to be acquired from multiple directions or in multiple planes. Although certain imaging systems 1304 are exemplified herein, it will be appreciated that any suitable imaging system may be selected by one of ordinary skill in the art.

Turning now to FIGS. 13A-13C, the surgical robotic system 100, 300, 600 relies on accurate positioning of the end-effector 112, 602, surgical instruments 608, and/or the patient 210 (e.g., patient tracking device 116) relative to the desired surgical area. In the embodiments shown in FIGS. 13A-13C, the tracking markers 118, 804 are rigidly attached to a portion of the instrument 608 and/or end-effector 112.

FIG. 13A depicts part of the surgical robotic system 100 with the robot 102 including base 106, robotic arm 104, and end-effector 112. The other elements, not illustrated, such as the display, cameras, etc. may also be present as described herein. FIG. 13B depicts a close-up view of the end-effector 112 with guide tube 114 and a plurality of tracking markers 118 rigidly affixed to the end-effector 112. In this embodiment, the plurality of tracking markers 118 are attached to the guide tube 112. FIG. 13C depicts an instrument 608 (in this case, a probe 608A) with a plurality of tracking markers 804 rigidly affixed to the instrument 608. As described elsewhere herein, the instrument 608 could include any suitable surgical instrument, such as, but not limited to, guide wire, cannula, a retractor, a drill, a reamer, a screw driver, an insertion tool, a removal tool, or the like.

When tracking an instrument 608, end-effector 112, or other object to be tracked in 3D, an array of tracking markers 118, 804 may be rigidly attached to a portion of the tool 608 or end-effector 112. Preferably, the tracking markers 118, 804 are attached such that the markers 118, 804 are out of the way (e.g., not impeding the surgical operation, visibility, etc.). The markers 118, 804 may be affixed to the instrument 608, end-effector 112, or other object to be tracked, for example, with an array 612. Usually three or four markers 118, 804 are used with an array 612. The array 612 may include a linear section, a cross piece, and may be asymmetric such that the markers 118, 804 are at different relative positions and locations with respect to one another. For example, as shown in FIG. 13C, a probe 608A with a 4-marker tracking array 612 is shown, and FIG. 13B depicts the end-effector 112 with a different 4-marker tracking array 612.

In FIG. 13C, the tracking array 612 functions as the handle 620 of the probe 608A. Thus, the four markers 804 are attached to the handle 620 of the probe 608A, which is out of the way of the shaft 622 and tip 624. Stereophotogrammetric tracking of these four markers 804 allows the instrument 608 to be tracked as a rigid body and for the tracking system 100, 300, 600 to precisely determine the position of the tip 624 and the orientation of the shaft 622 while the probe 608A is moved around in front of tracking cameras 200, 326.

To enable automatic tracking of one or more tools 608, end-effector 112, or other object to be tracked in 3D (e.g., multiple rigid bodies), the markers 118, 804 on each tool 608, end-effector 112, or the like, are arranged asymmetrically with a known inter-marker spacing. The reason for asymmetric alignment is so that it is unambiguous which marker 118, 804 corresponds to a particular location on the rigid body and whether markers 118, 804 are being viewed from the front or back, i.e., mirrored. For example, if the markers 118, 804 were arranged in a square on the tool 608 or end-effector 112, it would be unclear to the system 100, 300, 600 which marker 118, 804 corresponded to which corner of the square. For example, for the probe 608A, it would be unclear which marker 804 was closest to the shaft 622. Thus, it would be unknown which way the shaft 622 was extending from the array 612. Accordingly, each array 612 and thus each tool 608, end-effector 112, or other object to be tracked should have a unique marker pattern to allow it to be distinguished from other tools 608 or other objects being tracked. Asymmetry and unique marker patterns allow the system 100, 300, 600 to detect individual markers 118, 804 then to check the marker spacing against a stored template to determine which tool 608, end-effector 112, or other object they represent. Detected markers 118, 804 can then be sorted automatically and assigned to each tracked object in the correct order. Without this information, rigid body calculations could not then be performed to extract key geometric information, for example, such as tool tip 624 and alignment of the shaft 622, unless the user manually specified which detected marker 118, 804 corresponded to which position on each rigid body. These concepts are commonly known to those skilled in the methods of 3D optical tracking.

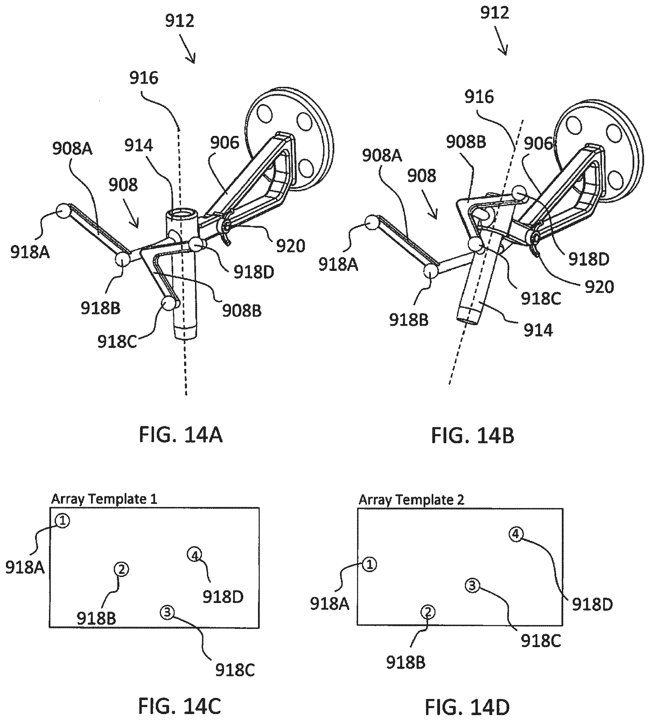

Turning now to FIGS. 14A-14D, an alternative version of an end-effector 912 with moveable tracking markers 918A-918D is shown. In FIG. 14A, an array with moveable tracking markers 918A-918D are shown in a first configuration, and in FIG. 14B the moveable tracking markers 918A-918D are shown in a second configuration, which is angled relative to the first configuration. FIG. 14C shows the template of the tracking markers 918A-918D, for example, as seen by the cameras 200, 326 in the first configuration of FIG. 14A; and FIG. 14D shows the template of tracking markers 918A-918D, for example, as seen by the cameras 200, 326 in the second configuration of FIG. 14B.

In this embodiment, 4-marker array tracking is contemplated wherein the markers 918A-918D are not all in fixed position relative to the rigid body and instead, one or more of the array markers 918A-918D can be adjusted, for example, during testing, to give updated information about the rigid body that is being tracked without disrupting the process for automatic detection and sorting of the tracked markers 918A-918D.

When tracking any tool, such as a guide tube 914 connected to the end-effector 912 of a robot system 100, 300, 600, the tracking array's primary purpose is to update the position of the end-effector 912 in the camera coordinate system. When using the rigid system, for example, as shown in FIG. 13B, the array 612 of reflective markers 118 rigidly extend from the guide tube 114. Because the tracking markers 118 are rigidly connected, knowledge of the marker locations in the camera coordinate system also provides exact location of the centerline, tip, and tail of the guide tube 114 in the camera coordinate system. Typically, information about the position of the end-effector 112 from such an array 612 and information about the location of a target trajectory from another tracked source are used to calculate the required moves that must be input for each axis of the robot 102 that will move the guide tube 114 into alignment with the trajectory and move the tip to a particular location along the trajectory vector.

Sometimes, the desired trajectory is in an awkward or unreachable location, but if the guide tube 114 could be swiveled, it could be reached. For example, a very steep trajectory pointing away from the base 106 of the robot 102 might be reachable if the guide tube 114 could be swiveled upward beyond the limit of the pitch (wrist up-down angle) axis, but might not be reachable if the guide tube 114 is attached parallel to the plate connecting it to the end of the wrist. To reach such a trajectory, the base 106 of the robot 102 might be moved or a different end-effector 112 with a different guide tube attachment might be exchanged with the working end-effector. Both of these solutions may be time consuming and cumbersome.

As best seen in FIGS. 14A and 14B, if the array 908 is configured such that one or more of the markers 918A-918D are not in a fixed position and instead, one or more of the markers 918A-918D can be adjusted, swiveled, pivoted, or moved, the robot 102 can provide updated information about the object being tracked without disrupting the detection and tracking process. For example, one of the markers 918A-918D may be fixed in position and the other markers 918A-918D may be moveable; two of the markers 918A-918D may be fixed in position and the other markers 918A-918D may be moveable; three of the markers 918A-918D may be fixed in position and the other marker 918A-918D may be moveable; or all of the markers 918A-918D may be moveable.

In the embodiment shown in FIGS. 14A and 14B, markers 918A, 918 B are rigidly connected directly to a base 906 of the end-effector 912, and markers 918C, 918D are rigidly connected to the tube 914. Similar to array 612, array 908 may be provided to attach the markers 918A-918D to the end-effector 912, instrument 608, or other object to be tracked. In this case, however, the array 908 is comprised of a plurality of separate components. For example, markers 918A, 918B may be connected to the base 906 with a first array 908A, and markers 918C, 918D may be connected to the guide tube 914 with a second array 908B. Marker 918A may be affixed to a first end of the first array 908A and marker 918B may be separated a linear distance and affixed to a second end of the first array 908A. While first array 908 is substantially linear, second array 908B has a bent or V-shaped configuration, with respective root ends, connected to the guide tube 914, and diverging therefrom to distal ends in a V-shape with marker 918C at one distal end and marker 918D at the other distal end. Although specific configurations are exemplified herein, it will be appreciated that other asymmetric designs including different numbers and types of arrays 908A, 908B and different arrangements, numbers, and types of markers 918A-918D are contemplated.

The guide tube 914 may be moveable, swivelable, or pivotable relative to the base 906, for example, across a hinge 920 or other connector to the base 906. Thus, markers 918C, 918D are moveable such that when the guide tube 914 pivots, swivels, or moves, markers 918C, 918D also pivot, swivel, or move. As best seen in FIG. 14A, guide tube 914 has a longitudinal axis 916 which is aligned in a substantially normal or vertical orientation such that markers 918A-918D have a first configuration. Turning now to FIG. 14B, the guide tube 914 is pivoted, swiveled, or moved such that the longitudinal axis 916 is now angled relative to the vertical orientation such that markers 918A-918D have a second configuration, different from the first configuration.

In contrast to the embodiment described for FIGS. 14A-14D, if a swivel existed between the guide tube 914 and the arm 104 (e.g., the wrist attachment) with all four markers 918A-918D remaining attached rigidly to the guide tube 914 and this swivel was adjusted by the user, the robotic system 100, 300, 600 would not be able to automatically detect that the guide tube 914 orientation had changed. The robotic system 100, 300, 600 would track the positions of the marker array 908 and would calculate incorrect robot axis moves assuming the guide tube 914 was attached to the wrist (the robotic arm 104) in the previous orientation. By keeping one or more markers 918A-918D (e.g., two markers 918C, 918D) rigidly on the tube 914 and one or more markers 918A-918D (e.g., two markers 918A, 918B) across the swivel, automatic detection of the new position becomes possible and correct robot moves are calculated based on the detection of a new tool or end-effector 112, 912 on the end of the robotic arm 104.

One or more of the markers 918A-918D are configured to be moved, pivoted, swiveled, or the like according to any suitable means. For example, the markers 918A-918D may be moved by a hinge 920, such as a clamp, spring, lever, slide, toggle, or the like, or any other suitable mechanism for moving the markers 918A-918D individually or in combination, moving the arrays 908A, 908B individually or in combination, moving any portion of the end-effector 912 relative to another portion, or moving any portion of the tool 608 relative to another portion.

As shown in FIGS. 14A and 14B, the array 908 and guide tube 914 may become reconfigurable by simply loosening the clamp or hinge 920, moving part of the array 908A, 908B relative to the other part 908A, 908B, and retightening the hinge 920 such that the guide tube 914 is oriented in a different position. For example, two markers 918C, 918D may be rigidly interconnected with the tube 914 and two markers 918A, 918B may be rigidly interconnected across the hinge 920 to the base 906 of the end-effector 912 that attaches to the robotic arm 104. The hinge 920 may be in the form of a clamp, such as a wing nut or the like, which can be loosened and retightened to allow the user to quickly switch between the first configuration (FIG. 14A) and the second configuration (FIG. 14B).

The cameras 200, 326 detect the markers 918A-918D, for example, in one of the templates identified in FIGS. 14C and 14D. If the array 908 is in the first configuration (FIG. 14A) and tracking cameras 200, 326 detect the markers 918A-918D, then the tracked markers match Array Template 1 as shown in FIG. 14C. If the array 908 is the second configuration (FIG. 14B) and tracking cameras 200, 326 detect the same markers 918A-918D, then the tracked markers match Array Template 2 as shown in FIG. 14D. Array Template 1 and Array Template 2 are recognized by the system 100, 300, 600 as two distinct tools, each with its own uniquely defined spatial relationship between guide tube 914, markers 918A-918D, and robot attachment. The user could therefore adjust the position of the end-effector 912 between the first and second configurations without notifying the system 100, 300, 600 of the change and the system 100, 300, 600 would appropriately adjust the movements of the robot 102 to stay on trajectory.

In this embodiment, there are two assembly positions in which the marker array matches unique templates that allow the system 100, 300, 600 to recognize the assembly as two different tools or two different end-effectors. In any position of the swivel between or outside of these two positions (namely, Array Template 1 and Array Template 2 shown in FIGS. 14C and 14D, respectively), the markers 918A-918D would not match any template and the system 100, 300, 600 would not detect any array present despite individual markers 918A-918D being detected by cameras 200, 326, with the result being the same as if the markers 918A-918D were temporarily blocked from view of the cameras 200, 326. It will be appreciated that other array templates may exist for other configurations, for example, identifying different instruments 608 or other end-effectors 112, 912, etc.

In the embodiment described, two discrete assembly positions are shown in FIGS. 14A and 14B. It will be appreciated, however, that there could be multiple discrete positions on a swivel joint, linear joint, combination of swivel and linear joints, pegboard, or other assembly where unique marker templates may be created by adjusting the position of one or more markers 918A-918D of the array relative to the others, with each discrete position matching a particular template and defining a unique tool 608 or end-effector 112, 912 with different known attributes. In addition, although exemplified for end-effector 912, it will be appreciated that moveable and fixed markers 918A-918D may be used with any suitable instrument 608 or other object to be tracked.

When using an external 3D tracking system 100, 300, 600 to track a full rigid body array of three or more markers attached to a robot's end-effector 112 (for example, as depicted in FIGS. 13A and 13B), it is possible to directly track or to calculate the 3D position of every section of the robot 102 in the coordinate system of the cameras 200, 326. The geometric orientations of joints relative to the tracker are known by design, and the linear or angular positions of joints are known from encoders for each motor of the robot 102, fully defining the 3D positions of all of the moving parts from the end-effector 112 to the base 116. Similarly, if a tracker were mounted on the base 106 of the robot 102 (not shown), it is likewise possible to track or calculate the 3D position of every section of the robot 102 from base 106 to end-effector 112 based on known joint geometry and joint positions from each motor's encoder.

In some situations, it may be desirable to track the positions of all segments of the robot 102 from fewer than three markers 118 rigidly attached to the end-effector 112. Specifically, if a tool 608 is introduced into the guide tube 114, it may be desirable to track full rigid body motion of the robot 902 with only one additional marker 118 being tracked.

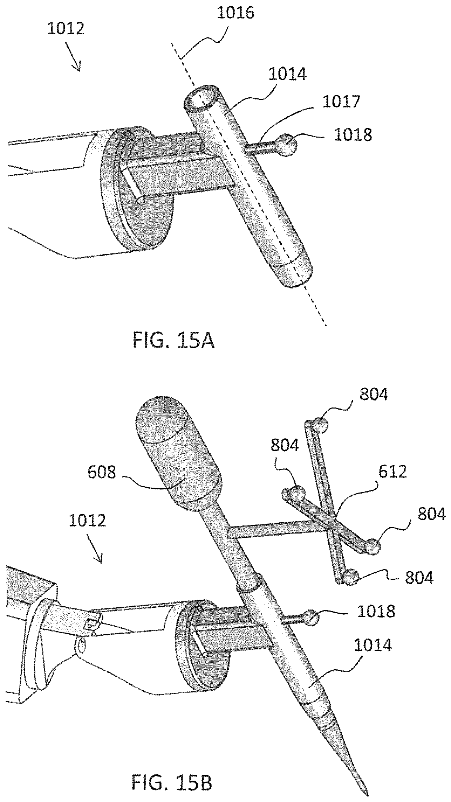

Turning now to FIGS. 15A-15E, an alternative version of an end-effector 1012 having only a single tracking marker 1018 is shown. End-effector 1012 may be similar to the other end-effectors described herein, and may include a guide tube 1014 extending along a longitudinal axis 1016. A single tracking marker 1018, similar to the other tracking markers described herein, may be rigidly affixed to the guide tube 1014. This single marker 1018 can serve the purpose of adding missing degrees of freedom to allow full rigid body tracking and/or can serve the purpose of acting as a surveillance marker to ensure that assumptions about robot and camera positioning are valid.

The single tracking marker 1018 may be attached to the robotic end-effector 1012 as a rigid extension to the end-effector 1012 that protrudes in any convenient direction and does not obstruct the surgeon's view. The tracking marker 1018 may be affixed to the guide tube 1014 or any other suitable location of on the end-effector 1012. When affixed to the guide tube 1014, the tracking marker 1018 may be positioned at a location between first and second ends of the guide tube 1014. For example, in FIG. 15A, the single tracking marker 1018 is shown as a reflective sphere mounted on the end of a narrow shaft 1017 that extends forward from the guide tube 1014 and is positioned longitudinally above a mid-point of the guide tube 1014 and below the entry of the guide tube 1014. This position allows the marker 1018 to be generally visible by cameras 200, 326 but also would not obstruct vision of the surgeon 120 or collide with other tools or objects in the vicinity of surgery. In addition, the guide tube 1014 with the marker 1018 in this position is designed for the marker array on any tool 608 introduced into the guide tube 1014 to be visible at the same time as the single marker 1018 on the guide tube 1014 is visible.

As shown in FIG. 15B, when a snugly fitting tool or instrument 608 is placed within the guide tube 1014, the instrument 608 becomes mechanically constrained in 4 of 6 degrees of freedom. That is, the instrument 608 cannot be rotated in any direction except about the longitudinal axis 1016 of the guide tube 1014 and the instrument 608 cannot be translated in any direction except along the longitudinal axis 1016 of the guide tube 1014. In other words, the instrument 608 can only be translated along and rotated about the centerline of the guide tube 1014. If two more parameters are known, such as (1) an angle of rotation about the longitudinal axis 1016 of the guide tube 1014; and (2) a position along the guide tube 1014, then the position of the end-effector 1012 in the camera coordinate system becomes fully defined.

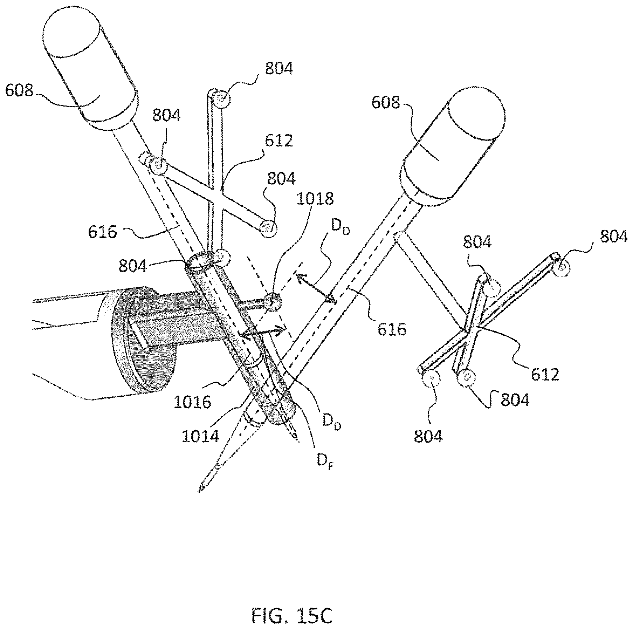

Referring now to FIG. 15C, the system 100, 300, 600 should be able to know when a tool 608 is actually positioned inside of the guide tube 1014 and is not instead outside of the guide tube 1014 and just somewhere in view of the cameras 200, 326. The tool 608 has a longitudinal axis or centerline 616 and an array 612 with a plurality of tracked markers 804. The rigid body calculations may be used to determine where the centerline 616 of the tool 608 is located in the camera coordinate system based on the tracked position of the array 612 on the tool 608.

The fixed normal (perpendicular) distance DF from the single marker 1018 to the centerline or longitudinal axis 1016 of the guide tube 1014 is fixed and is known geometrically, and the position of the single marker 1018 can be tracked. Therefore, when a detected distance DD from tool centerline 616 to single marker 1018 matches the known fixed distance DF from the guide tube centerline 1016 to the single marker 1018, it can be determined that the tool 608 is either within the guide tube 1014 (centerlines 616, 1016 of tool 608 and guide tube 1014 coincident) or happens to be at some point in the locus of possible positions where this distance DD matches the fixed distance DF. For example, in FIG. 15C, the normal detected distance DD from tool centerline 616 to the single marker 1018 matches the fixed distance DF from guide tube centerline 1016 to the single marker 1018 in both frames of data (tracked marker coordinates) represented by the transparent tool 608 in two positions, and thus, additional considerations may be needed to determine when the tool 608 is located in the guide tube 1014.

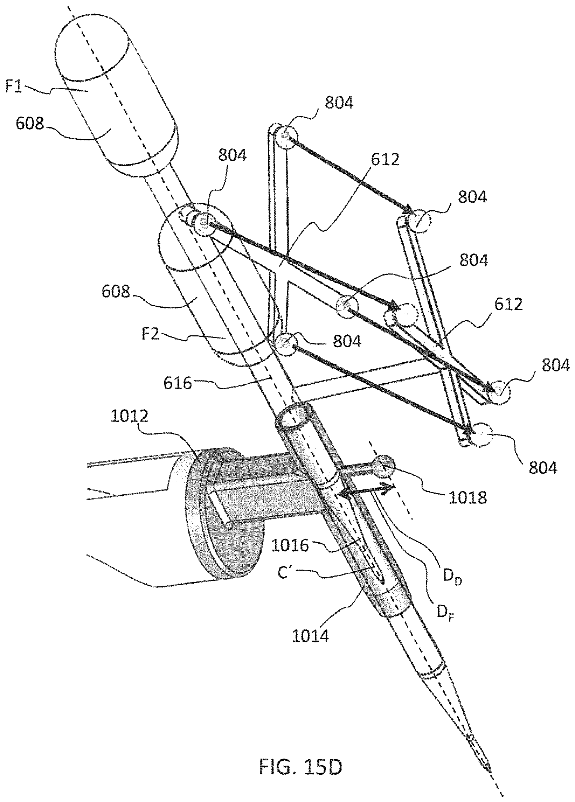

Turning now to FIG. 15D, programmed logic can be used to look for frames of tracking data in which the detected distance DD from tool centerline 616 to single marker 1018 remains fixed at the correct length despite the tool 608 moving in space by more than some minimum distance relative to the single sphere 1018 to satisfy the condition that the tool 608 is moving within the guide tube 1014. For example, a first frame F1 may be detected with the tool 608 in a first position and a second frame F2 may be detected with the tool 608 in a second position (namely, moved linearly with respect to the first position). The markers 804 on the tool array 612 may move by more than a given amount (e.g., more than 5mm total) from the first frame F1 to the second frame F2. Even with this movement, the detected distance DD from the tool centerline vector C' to the single marker 1018 is substantially identical in both the first frame F1 and the second frame F2.

Logistically, the surgeon 120 or user could place the tool 608 within the guide tube 1014 and slightly rotate it or slide it down into the guide tube 1014 and the system 100, 300, 600 would be able to detect that the tool 608 is within the guide tube 1014 from tracking of the five markers (four markers 804 on tool 608 plus single marker 1018 on guide tube 1014). Knowing that the tool 608 is within the guide tube 1014, all 6 degrees of freedom may be calculated that define the position and orientation of the robotic end-effector 1012 in space. Without the single marker 1018, even if it is known with certainty that the tool 608 is within the guide tube 1014, it is unknown where the guide tube 1014 is located along the tool's centerline vector C' and how the guide tube 1014 is rotated relative to the centerline vector C'.

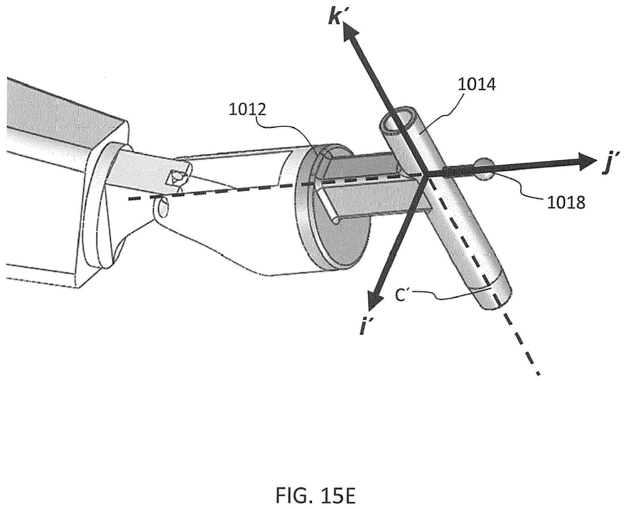

With emphasis on FIG. 15E, the presence of the single marker 1018 being tracked as well as the four markers 804 on the tool 608, it is possible to construct the centerline vector C' of the guide tube 1014 and tool 608 and the normal vector through the single marker 1018 and through the centerline vector C'. This normal vector has an orientation that is in a known orientation relative to the forearm of the robot distal to the wrist (in this example, oriented parallel to that segment) and intersects the centerline vector C' at a specific fixed position. For convenience, three mutually orthogonal vectors k', j', i' can be constructed, as shown in FIG. 15E, defining rigid body position and orientation of the guide tube 1014. One of the three mutually orthogonal vectors k' is constructed from the centerline vector C', the second vector j' is constructed from the normal vector through the single marker 1018, and the third vector i' is the vector cross product of the first and second vectors k', j'. The robot's joint positions relative to these vectors k', j', i' are known and fixed when all joints are at zero, and therefore rigid body calculations can be used to determine the location of any section of the robot relative to these vectors k', j', i' when the robot is at a home position. During robot movement, if the positions of the tool markers 804 (while the tool 608 is in the guide tube 1014) and the position of the single marker 1018 are detected from the tracking system, and angles/linear positions of each joint are known from encoders, then position and orientation of any section of the robot can be determined.

In some embodiments, it may be useful to fix the orientation of the tool 608 relative to the guide tube 1014. For example, the end-effector guide tube 1014 may be oriented in a particular position about its axis 1016 to allow machining or implant positioning. Although the orientation of anything attached to the tool 608 inserted into the guide tube 1014 is known from the tracked markers 804 on the tool 608, the rotational orientation of the guide tube 1014 itself in the camera coordinate system is unknown without the additional tracking marker 1018 (or multiple tracking markers in other embodiments) on the guide tube 1014. This marker 1018 provides essentially a "clock position" from -180° to +180° based on the orientation of the marker 1018 relative to the centerline vector C'. Thus, the single marker 1018 can provide additional degrees of freedom to allow full rigid body tracking and/or can act as a surveillance marker to ensure that assumptions about the robot and camera positioning are valid.

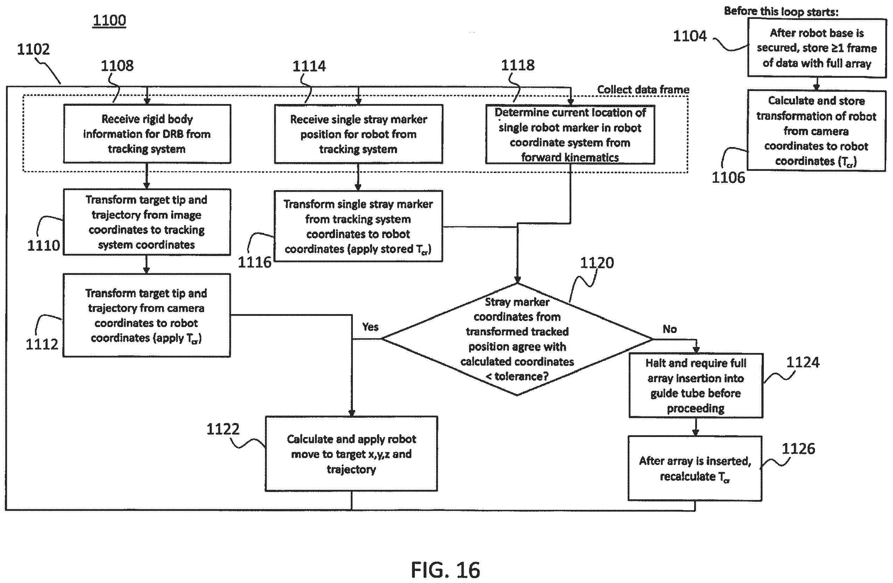

FIG. 16 is a block diagram of a method 1100 for navigating and moving the end-effector 1012 (or any other end-effector described herein) of the robot 102 to a desired target trajectory. Another use of the single marker 1018 on the robotic end-effector 1012 or guide tube 1014 is as part of the method 1100 enabling the automated safe movement of the robot 102 without a full tracking array attached to the robot 102. This method 1100 functions when the tracking cameras 200, 326 do not move relative to the robot 102 (i.e., they are in a fixed position), the tracking system's coordinate system and robot's coordinate system are co-registered, and the robot 102 is calibrated such that the position and orientation of the guide tube 1014 can be accurately determined in the robot's Cartesian coordinate system based only on the encoded positions of each robotic axis.

For this method 1100, the coordinate systems of the tracker and the robot must be co-registered, meaning that the coordinate transformation from the tracking system's Cartesian coordinate system to the robot's Cartesian coordinate system is needed. For convenience, this coordinate transformation can be a 4x4 matrix of translations and rotations that is well known in the field of robotics. This transformation will be termed Tcr to refer to "transformation - camera to robot". Once this transformation is known, any new frame of tracking data, which is received as x,y,z coordinates in vector form for each tracked marker, can be multiplied by the 4x4 matrix and the resulting x,y,z coordinates will be in the robot's coordinate system. To obtain Tcr, a full tracking array on the robot is tracked while it is rigidly attached to the robot at a location that is known in the robot's coordinate system, then known rigid body methods are used to calculate the transformation of coordinates. It should be evident that any tool 608 inserted into the guide tube 1014 of the robot 102 can provide the same rigid body information as a rigidly attached array when the additional marker 1018 is also read. That is, the tool 608 need only be inserted to any position within the guide tube 1014 and at any rotation within the guide tube 1014, not to a fixed position and orientation. Thus, it is possible to determine Tcr by inserting any tool 608 with a tracking array 612 into the guide tube 1014 and reading the tool's array 612 plus the single marker 1018 of the guide tube 1014 while at the same time determining from the encoders on each axis the current location of the guide tube 1014 in the robot's coordinate system.

Logic for navigating and moving the robot 102 to a target trajectory is provided in the method 1100 of FIG. 16. Before entering the loop 1102, it is assumed that the transformation Tcr was previously stored. Thus, before entering loop 1102, in step 1104, after the robot base 106 is secured, greater than or equal to one frame of tracking data of a tool inserted in the guide tube while the robot is static is stored; and in step 1106, the transformation of robot guide tube position from camera coordinates to robot coordinates Tcr is calculated from this static data and previous calibration data. Tcr should remain valid as long as the cameras 200, 326 do not move relative to the robot 102. If the cameras 200, 326 move relative to the robot 102, and Tcr needs to be re-obtained, the system 100, 300, 600 can be made to prompt the user to insert a tool 608 into the guide tube 1014 and then automatically perform the necessary calculations.

In the flowchart of method 1100, each frame of data collected consists of the tracked position of the DRB 1404 on the patient 210, the tracked position of the single marker 1018 on the end-effector 1014, and a snapshot of the positions of each robotic axis. From the positions of the robot's axes, the location of the single marker 1018 on the end-effector 1012 is calculated. This calculated position is compared to the actual position of the marker 1018 as recorded from the tracking system. If the values agree, it can be assured that the robot 102 is in a known location. The transformation Tcr is applied to the tracked position of the DRB 1404 so that the target for the robot 102 can be provided in terms of the robot's coordinate system. The robot 102 can then be commanded to move to reach the target.

After steps 1104, 1106, loop 1102 includes step 1108 receiving rigid body information for DRB 1404 from the tracking system; step 1110 transforming target tip and trajectory from image coordinates to tracking system coordinates; and step 1112 transforming target tip and trajectory from camera coordinates to robot coordinates (apply Tcr). Loop 1102 further includes step 1114 receiving a single stray marker position for robot from tracking system; and step 1116 transforming the single stray marker from tracking system coordinates to robot coordinates (apply stored Tcr). Loop 1102 also includes step 1118 determining current location of the single robot marker 1018 in the robot coordinate system from forward kinematics. The information from steps 1116 and 1118 is used to determine step 1120 whether the stray marker coordinates from transformed tracked position agree with the calculated coordinates being less than a given tolerance. If yes, proceed to step 1122, calculate and apply robot move to target x, y, z and trajectory. If no, proceed to step 1124, halt and require full array insertion into guide tube 1014 before proceeding; step 1126 after array is inserted, recalculate Tcr; and then proceed to repeat steps 1108, 1114, and 1118.

This method 1100 has advantages over a method in which the continuous monitoring of the single marker 1018 to verify the location is omitted. Without the single marker 1018, it would still be possible to determine the position of the end-effector 1012 using Tcr and to send the end-effector 1012 to a target location but it would not be possible to verify that the robot 102 was actually in the expected location. For example, if the cameras 200, 326 had been bumped and Tcr was no longer valid, the robot 102 would move to an erroneous location. For this reason, the single marker 1018 provides value with regard to safety.

For a given fixed position of the robot 102, it is theoretically possible to move the tracking cameras 200, 326 to a new location in which the single tracked marker 1018 remains unmoved since it is a single point, not an array. In such a case, the system 100, 300, 600 would not detect any error since there would be agreement in the calculated and tracked locations of the single marker 1018. However, once the robot's axes caused the guide tube 1012 to move to a new location, the calculated and tracked positions would disagree and the safety check would be effective.

The term "surveillance marker" may be used, for example, in reference to a single marker that is in a fixed location relative to the DRB 1404. In this instance, if the DRB 1404 is bumped or otherwise dislodged, the relative location of the surveillance marker changes and the surgeon 120 can be alerted that there may be a problem with navigation. Similarly, in the embodiments described herein, with a single marker 1018 on the robot's guide tube 1014, the system 100, 300, 600 can continuously check whether the cameras 200, 326 have moved relative to the robot 102. If registration of the tracking system's coordinate system to the robot's coordinate system is lost, such as by cameras 200, 326 being bumped or malfunctioning or by the robot malfunctioning, the system 100, 300, 600 can alert the user and corrections can be made. Thus, this single marker 1018 can also be thought of as a surveillance marker for the robot 102.

It should be clear that with a full array permanently mounted on the robot 102 (e.g., the plurality of tracking markers 702 on end-effector 602 shown in FIGS. 7A-7C) such functionality of a single marker 1018 as a robot surveillance marker is not needed because it is not required that the cameras 200, 326 be in a fixed position relative to the robot 102, and Tcr is updated at each frame based on the tracked position of the robot 102. Reasons to use a single marker 1018 instead of a full array are that the full array is more bulky and obtrusive, thereby blocking the surgeon's view and access to the surgical field 208 more than a single marker 1018, and line of sight to a full array is more easily blocked than line of sight to a single marker 1018.

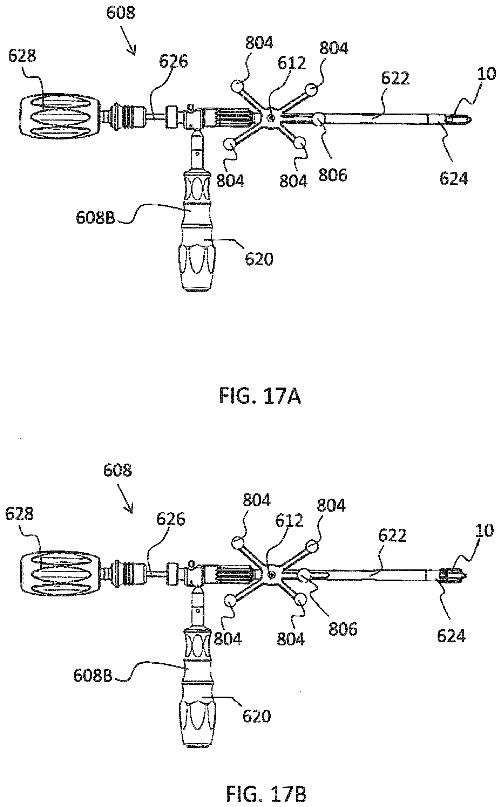

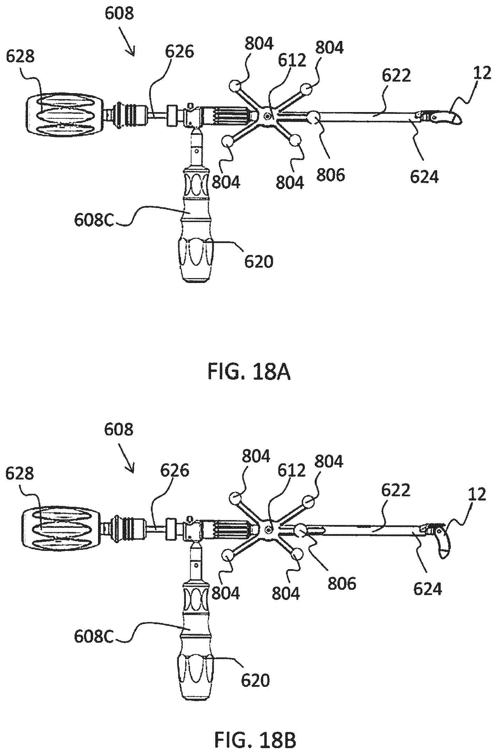

Turning now to FIGS. 17A-17B and 18A-18B, instruments 608, such as implant holders 608B, 608C, are depicted which include both fixed and moveable tracking markers 804, 806. The implant holders 608B, 608C may have a handle 620 and an outer shaft 622 extending from the handle 620. The shaft 622 may be positioned substantially perpendicular to the handle 620, as shown, or in any other suitable orientation. An inner shaft 626 may extend through the outer shaft 622 with a knob 628 at one end. Implant 10, 12 connects to the shaft 622, at the other end, at tip 624 of the implant holder 608B, 608C using typical connection mechanisms known to those of skill in the art. The knob 628 may be rotated, for example, to expand or articulate the implant 10, 12. U.S. Patent Nos. 8,709,086 and 8,491,659, which are incorporated by reference herein, describe expandable fusion devices and methods of installation.

When tracking the tool 608, such as implant holder 608B, 608C, the tracking array 612 may contain a combination of fixed markers 804 and one or more moveable markers 806 which make up the array 612 or is otherwise attached to the implant holder 608B, 608C. The navigation array 612 may include at least one or more (e.g., at least two) fixed position markers 804, which are positioned with a known location relative to the implant holder instrument 608B, 608C. These fixed markers 804 would not be able to move in any orientation relative to the instrument geometry and would be useful in defining where the instrument 608 is in space. In addition, at least one marker 806 is present which can be attached to the array 612 or the instrument itself which is capable of moving within a pre-determined boundary (e.g., sliding, rotating, etc.) relative to the fixed markers 804. The system 100, 300, 600 (e.g., the software) correlates the position of the moveable marker 806 to a particular position, orientation, or other attribute of the implant 10 (such as height of an expandable interbody spacer shown in FIGS. 17A-17B or angle of an articulating interbody spacer shown in FIGS. 18A-18B). Thus, the system and/or the user can determine the height or angle of the implant 10, 12 based on the location of the moveable marker 806.

In the embodiment shown in FIGS. 17A-17B, four fixed markers 804 are used to define the implant holder 608B and a fifth moveable marker 806 is able to slide within a pre-determined path to provide feedback on the implant height (e.g., a contracted position or an expanded position). FIG. 17A shows the expandable spacer 10 at its initial height, and FIG. 17B shows the spacer 10 in the expanded state with the moveable marker 806 translated to a different position. In this case, the moveable marker 806 moves closer to the fixed markers 804 when the implant 10 is expanded, although it is contemplated that this movement may be reversed or otherwise different. The amount of linear translation of the marker 806 would correspond to the height of the implant 10. Although only two positions are shown, it would be possible to have this as a continuous function whereby any given expansion height could be correlated to a specific position of the moveable marker 806.

Turning now to FIGS. 18A-18B, four fixed markers 804 are used to define the implant holder 608C and a fifth, moveable marker 806 is configured to slide within a pre-determined path to provide feedback on the implant articulation angle. FIG. 18A shows the articulating spacer 12 at its initial linear state, and FIG. 18B shows the spacer 12 in an articulated state at some offset angle with the moveable marker 806 translated to a different position. The amount of linear translation of the marker 806 would correspond to the articulation angle of the implant 12. Although only two positions are shown, it would be possible to have this as a continuous function whereby any given articulation angle could be correlated to a specific position of the moveable marker 806.

In these embodiments, the moveable marker 806 slides continuously to provide feedback about an attribute of the implant 10, 12 based on position. It is also contemplated that there may be discreet positions that the moveable marker 806 must be in which would also be able to provide further information about an implant attribute. In this case, each discreet configuration of all markers 804, 806 correlates to a specific geometry of the implant holder 608B, 608C and the implant 10, 12 in a specific orientation or at a specific height. In addition, any motion of the moveable marker 806 could be used for other variable attributes of any other type of navigated implant.

Although depicted and described with respect to linear movement of the moveable marker 806, the moveable marker 806 should not be limited to just sliding as there may be applications where rotation of the marker 806 or other movements could be useful to provide information about the implant 10, 12. Any relative change in position between the set of fixed markers 804 and the moveable marker 806 could be relevant information for the implant 10, 12 or other device. In addition, although expandable and articulating implants 10, 12 are exemplified, the instrument 608 could work with other medical devices and materials, such as spacers, cages, plates, fasteners, nails, screws, rods, pins, wire structures, sutures, anchor clips, staples, stents, bone grafts, biologics, cements, or the like.

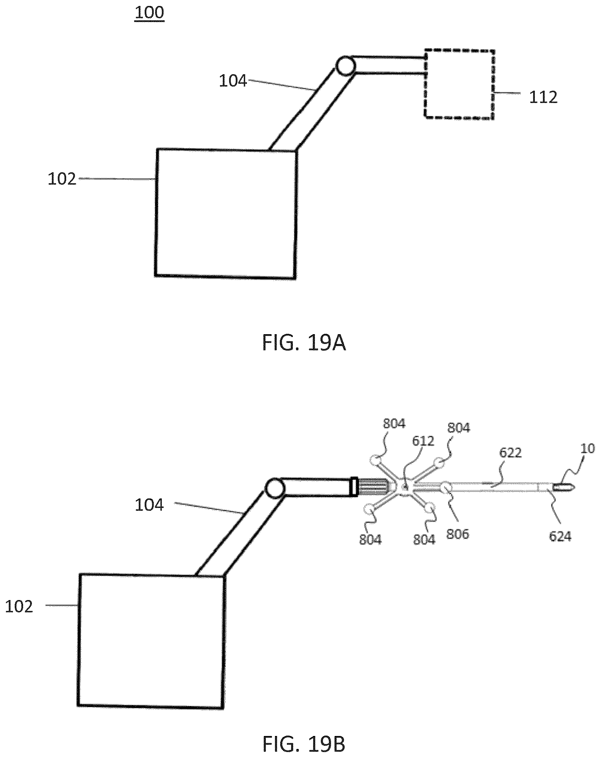

Turning now to FIG. 19A, it is envisioned that the robot end-effector 112 is interchangeable with other types of end-effectors 112. Moreover, it is contemplated that each end-effector 112 may be able to perform one or more functions based on a desired surgical procedure. For example, the end-effector 112 having a guide tube 114 may be used for guiding an instrument 608 as described herein. In addition, end-effector 112 may be replaced with a different or alternative end-effector 112 that controls a surgical device, instrument, or implant, for example.

The alternative end-effector 112 may include one or more devices or instruments coupled to and controllable by the robot. By way of non-limiting example, the end-effector 112, as depicted in FIG. 19A, may comprise a retractor (for example, one or more retractors disclosed in U.S. Patent Nos. 8,992,425 and 8,968,363) or one or more mechanisms for inserting or installing surgical devices such as expandable intervertebral fusion devices (such as expandable implants exemplified in U.S. Patent Nos. 8,845,734; 9,510,954; and 9,456,903), stand-alone intervertebral fusion devices (such as implants exemplified in U.S. Patent Nos. 9,364,343 and 9,480,579), expandable corpectomy devices (such as corpectomy implants exemplified in U.S. Patent Nos. 9,393,128 and 9,173,747), articulating spacers (such as implants exemplified in U.S. Patent No. 9,259,327), facet prostheses (such as devices exemplified in U.S. Patent No. 9,539,031), laminoplasty devices (such as devices exemplified in U.S. Patent No. 9,486,253), spinous process spacers (such as implants exemplified in U.S. Patent No. 9,592,082), inflatables, fasteners including polyaxial screws, uniplanar screws, pedicle screws, posted screws, and the like, bone fixation plates, rod constructs and revision devices (such as devices exemplified in U.S. Patent No. 8,882,803), artificial and natural discs, motion preserving devices and implants, spinal cord stimulators (such as devices exemplified in U.S. Patent No. 9,440,076), and other surgical devices. The end-effector 112 may include one or instruments directly or indirectly coupled to the robot for providing bone cement, bone grafts, living cells, pharmaceuticals, or other deliverable to a surgical target. The end-effector 112 may also include one or more instruments designed for performing a discectomy, kyphoplasty, vertebrostenting, dilation, or other surgical procedure.

The end-effector itself and/or the implant, device, or instrument may include one or more markers 118 such that the location and position of the markers 118 may be identified in three-dimensions. It is contemplated that the markers 118 may include active or passive markers 118, as described herein, that may be directly or indirectly visible to the cameras 200. Thus, one or more markers 118 located on an implant 10, for example, may provide for tracking of the implant 10 before, during, and after implantation.

As shown in FIG. 19B, the end-effector 112 may include an instrument 608 or portion thereof that is coupled to the robotic arm 104 (for example, the instrument 608 may be coupled to the robotic arm 104 by the coupling mechanism shown in FIGS. 9A-9C) and is controllable by the robot system 100. Thus, in the embodiment shown in FIG. 19B, the robot system 100 is able to insert implant 10 into a patient and expand or contract the expandable implant 10. Accordingly, the robot system 100 may be configured to assist a surgeon or to operate partially or completely independently thereof. Thus, it is envisioned that the robot system 100 may be capable of controlling each alternative end-effector 112 for its specified function or surgical procedure.

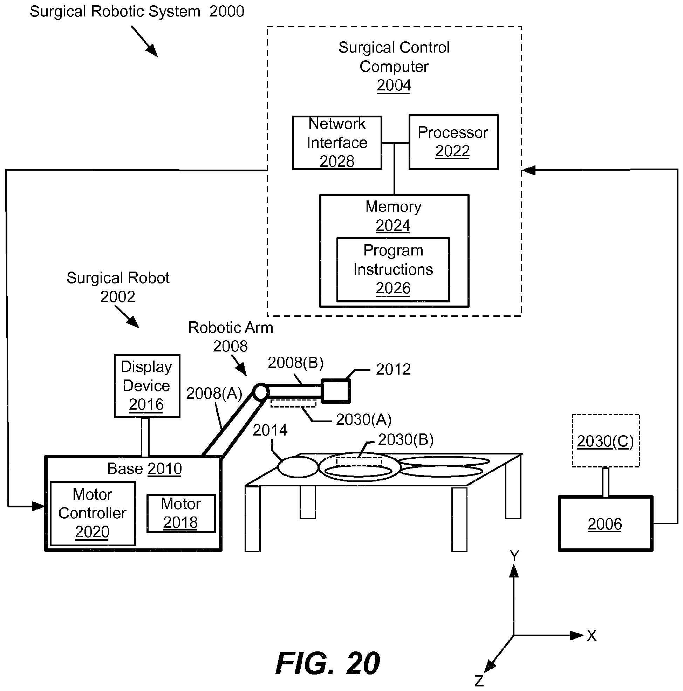

As discussed above, surgical robotic systems, such as the surgical robotic system 100, are described in exemplary embodiments disclosed herein. In this regard, FIG. 20 illustrates a block diagram of an exemplary surgical robotic system 2000. The surgical robotic system 2000 includes a surgical robot 2002, a surgical control computer 2004, and a sensor processing computer 2006. The surgical robot 2002 includes a robotic arm 2008 having an upper portion 2008(A) and a lower portion 2008(B) connected via a hinging mechanism. The upper portion 2008(A) of the robotic arm 2008 is connected to a base 2010 of the surgical robot 2002, and the lower portion 2008(B) is connected to a surgical end-effector 2012. The surgical end-effector 2012 interacts with various aspects of the environment so as to assist in or perform surgery on a patient 2014. A display device 2016 may be connected to the base 2010 of the surgical robot 2002 to provide information to a surgeon and/or an operator during surgery. The base 2010 includes a motor 2018 connected to move the robotic arm 2008 via a motor controller 2020 in response to commands from the surgical control computer 2004. The surgical control computer 2004 includes a processor 2022, a memory 2024 including program instructions 2026, and a network interface 2028. The processor 2022 of the surgical control computer 2004 receives sensed data, such as a proximity signal (discussed in further detail below), from a sensor or sensor(s) 2030(A)-2030(C) via the sensor processing computer 2006. As illustrated in FIG. 20, and discussed in greater detail below, the sensor or sensor(s) 2030(A)-2030(C) may be organized in various configurations across different sensor locations, as indicated by the dotted-line boxes and may operate with various functions discussed below.

During a surgery using the surgical robotic system 2000, the surgical robot 2002 may assist in or perform surgical operations on or near the patient 2014. The robotic arm 2008 of the surgical robot 2002 may be moved by the surgeon and/or operator, or by an autonomous control computer, to position the surgical end-effector 2012 relative to the patient 2014. While the robotic arm 2008 is positioned adjacent to the patient 2014, the sensor or sensor(s) 2030(A)-2030(C) (described in more detail below) may send sensed data, such as a proximity signal indicating proximity of the robotic arm 2008 to the patient 2014, to the surgical control computer 2004 via the sensor processing computer 2006. In response to receiving the proximity signal(s) from the sensor processing computer 2006, the processor 2022 performs operations of the program instructions 2026 stored in the memory 2024 of the surgical control computer 2004. The processor 2022 determines if or when the robotic arm 2008 has collided with the patient 2014 or is predicted to collide with the patient 2014 based on the received proximity signal(s). The processor 2022, via the network interface 2028, then performs a remedial action responsive to the determination. For example, the processor 2022 may perform a remedial action by controlling the display device 2016 to display a collision warning to the surgeon and/or operator so that the surgeon and/or operator may avoid a collision of the robotic arm 2008 with a patient. This embodiment may be particularly useful in manually-controlled surgical robotic systems. In another example, the processor 2022 performs a remedial action by sending a command to the motor controller 2020 instructing the motor controller 2020 to inhibit movement of the robotic arm 2008 in a further direction toward the patient 2014.

By having the surgical robotic system 2000 perform remedial action(s) responsive to determining an actual or predicted collision, collisions between the robotic arm 2008 and the patient 2014 can be reduced and/or eliminated without the need for increased surgeon oversight and/or excessive range of motion restrictions for the robotic arm 2008. Surgical robotic systems may thereby reduce and/or eliminate the consequences of collisions, which can include tissue damage, blood loss, and scarring, and may allow more confident movement of the robotic arm and increased range of motion which can result in shorter operation times and reduced surgery cost.

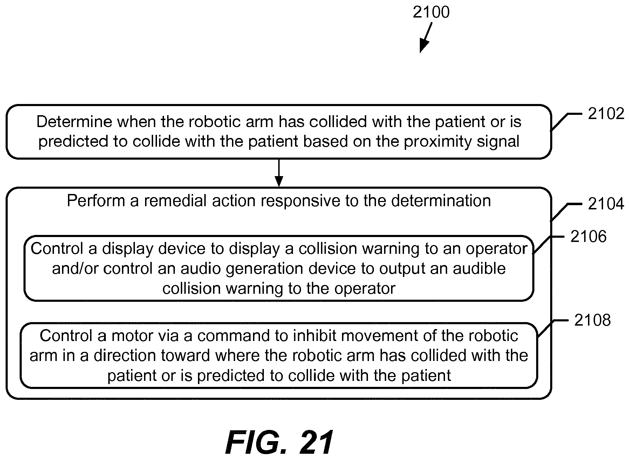

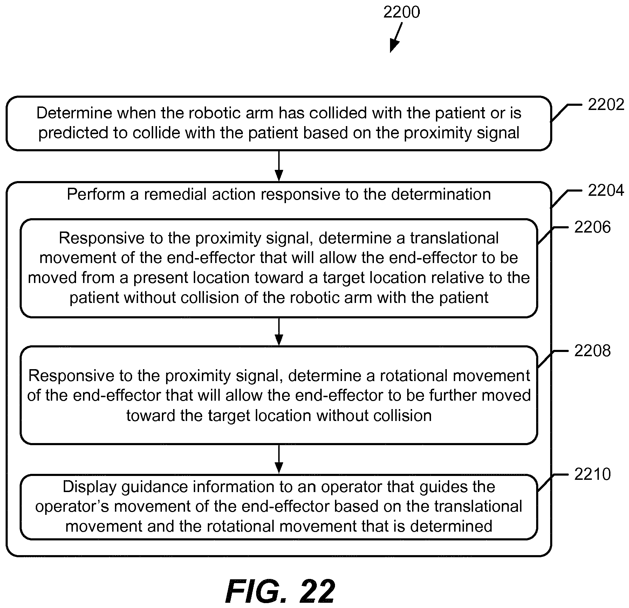

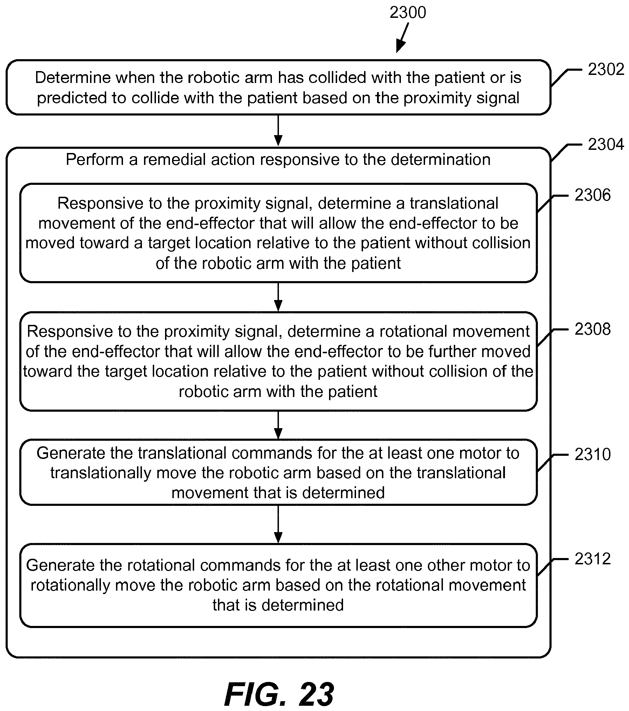

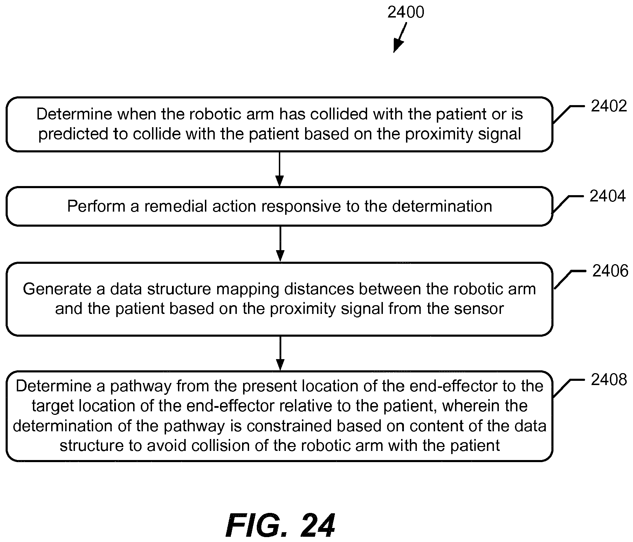

In this regard, FIGS. 21-24 illustrate flowcharts 2100, 2200, 2300, and 2400 showing exemplary operations that may be provided by the program instructions 2026 stored in the memory 2024 and executed by the processor 2022. Each exemplary flowchart 2100, 2200, 2300, and 2400 includes the operations discussed above of determining when the robotic arm 2008 has collided with the patient 2014 or is predicted to collide with the patient 2014 based on the proximity signal (see block 2102 in FIG. 21, block 2202 in FIG. 22, block 2302 in FIG. 23, and block 2402 in FIG. 24) and performing a remedial action responsive to the determination (see block 2104 in FIG. 21, block 2204 in FIG. 22, block 2304 in FIG. 23, and block 2404 in FIG. 24). However, each flowchart 2100, 2200, 2300, and 2400 is distinct in that each is directed to a different embodiment showing more specific and/or additional operations. Specifically, the flowcharts 2100, 2200, and 2300 in FIGS. 21-23 are each directed to a set of more specific operations associated with performing remedial action(s) responsive to the determination, as disclosed in blocks 2104, 2204, and 2304. FIG. 24 is distinct from FIGS. 21-23 in that the flowchart 2400 is further directed to additional operations beyond determining when the robotic arm has collided with the patient or is predicted to collide with the patient based on the proximity signal, and performing a remedial action responsive to the determination.

Although these embodiments may be presented as distinct from one another in the examples discussed herein, they may also be combined in a multitude of ways, so long as such a combination is or would be operable. Furthermore, although the operations in the embodiments disclosed herein may be presented as occurring in a given combination, each operation in a given embodiment may be performed separately or in combination with operations of other embodiments, so long as such a combination of operations is or would be operable. In any such manner, by executing the operations disclosed in FIGS. 21-24, the processor 2022 is able to perform remedial action(s) in the surgical robotic system 2000 in response to an actual or predicted collision of the robotic arm 2008, in accordance with some exemplary embodiments.

Referring to FIG. 21 and the surgical robotic system 2000 of FIG. 20, performing a remedial action can include controlling a display device to display a collision warning to an operator and/or controlling an audio generation device to output an audible collision warning to the operator (block 2106 of FIG. 21). By providing a collision warning in such a manner, the operator and/or surgeon can stop or inhibit the movement of the robotic arm 2008 to avoid a collision of the robotic arm 2008 with the patient 2014. In some examples, the display device is the display device 2016, which may be a video monitor, projector, or other screen for viewing feedback of the surgical robotic system 2000. In additional examples, the audio generation device may be incorporated into the display device 2016, or elsewhere in the surgical robotic system 2000, such as in the surgical control computer 2004 or alone in a separate audio generation unit.

While displaying and/or outputting a visible/audible collision warning may be particularly beneficial in a manually-controlled surgical robotic system, such collision warnings may also be beneficial in autonomously-controlled surgical robotic systems. For example, an autonomously-controlled surgical robotic system performing a surgery without manual operation provided by a human operator may benefit from such a collision warning because a different, autonomous operator unit can be alerted of an actual or predicted collision of the robotic arm 2008 with the patient 2014 and can perform additional remedial action(s) as desired. In this regard, different surgical robotic systems may be able to be combined to provide the same benefits across systems as discussed herein. Furthermore, such a collision warning may be beneficial in autonomously-controlled surgical robotic systems that are monitored by a human because it may alert the monitor and/or human of such a collision so that additional remedial action(s) can be performed.

It should also be appreciated that the display device or the audio generation device can be located at various places. For example, if the surgery is a remote surgery where the operator is not located in the same place as the surgical robot 2008, the display device and/or the audio generation device may be located at a remote location. In this manner, the collision warning may still be visible and/or audible to the operator and/or surgeon so the operator and/or surgeon can take additional remedial action(s). Furthermore, while embodiments disclosed herein contemplate collisions between a robotic arm and a patient, collisions eligible for sensing and remedial actions are not limited to robotic arm and patient collisions. Rather, embodiments disclosed herein may also be applied to collisions between a robotic arm and almost any other object, including an operating table, a sensor, the robotic arm itself, a surgical control computer, a sensor processing computer, a surgeon and/or an operator, or any other device or feature included in an operating environment.

With further reference to FIG. 21, performing a remedial action can also include controlling a motor or combination of motors via a command to inhibit movement of the robotic arm in a direction toward where the robotic arm has collided with the patient or is predicted to collide with the patient (block 2108 of FIG. 21). For example, in a surgery where the end-effector 2012 is to be positioned on the left side of the abdomen of the patient 2014, the robotic arm 2008 may need to move along the Z-axis toward the patient 2014 to position the end-effector 2012 as desired. However, in moving along the Z-axis, the robotic arm 2008 may collide with, or be predicted to collide with, the patient 2014. In determining such an actual or potential collision, the processor 2022 may send a command to the motor controller 2020 to cause the motor 2018 to inhibit and/or stop movement of the robotic arm 2008 in the direction toward where the robotic arm 2008 has collided or is predicted to collide with the patient 2014. For example, if the robotic arm 2008 has collided with the left shoulder of the patient 2014, the processor 2022 may send a command to the motor controller 2020 causing the motor 2018 to stop movement of the robotic arm 2008 in the positive Z-axis direction (i.e., out of the page). While the robotic arm 2008 may, in some embodiments, move in the X-axis direction, the Y-axis direction, and/or the negative Z-axis direction, to move away from the actual or predicted collision location (as discussed below), the robotic arm 2008 may cease movement in the positive Z-axis direction in this example so as to stop causing potentially greater harm. In this manner, remedial action(s) performed in the surgical robotic system 2000 in response to an actual or predicted collision of the robotic arm 2008 may reduce greater harm, thereby allowing for reduced tissue damage, blood loss, and scarring, as well as shorter operation times and healing times, at a reduced cost.

With regard to FIG. 22, and with further reference to the surgical robotic system 2000 of FIG. 20, performing a remedial action as discussed above can also include, responsive to the proximity signal, determining a translational movement of the end-effector that will allow the end-effector to be moved from the present location toward a target location relative to the patient without collision of the robotic arm with the patient (block 2206 of FIG. 22). Similarly, performing such a remedial action can further include, responsive to the proximity signal, determining a rotational movement of the end-effector that will allow the end-effector to be further moved toward the target location without collision (block 2208 in FIG. 22). Once determined, guidance information may be displayed to an operator that guides the operator's movement of the end-effector based on the translational movement and the rotational movement that is determined (block 2210 in FIG. 22).

For example, with respect to FIG. 20, in a surgery where the end-effector 2012 is being moved downward along the Y-axis toward the patient 2014, the processor 2022 may determine, based on the proximity signal(s) captured by the sensor(s) 2030(A)-2030(C) and transmitted by the sensor processing computer 2006 to the processor 2022, that the robotic arm 2008 will soon collide with the patient 2014. In response to this determination, the processor 2022 may determine that the robotic arm 2008 should be moved in the positive Z-axis direction to avoid such a collision. However, to still position the end-effector 2012 as desired, the processor 2022 may further determine that the end-effector 2012 should be rotated to be moved into a target location without collision. To assist the surgeon and/or operator in performing such translational and rotational movements, either in sequence or simultaneously, the display device 2016 may display guidance information to the operator that guides the operator's movement of the end-effector 2012 in accordance with the determined motions.

In additional embodiments, the surgical control computer 2004 may also determine a new position for the base 2010 of the surgical robot 2002 such that the robotic arm 2008 can approach the patient 2014 with a less-problematic set of joint angles for a given position of the end-effector 2012. For example, it may be desirable to provide an increased roll angle at the expense of a decreased pitch angle of the robotic arm 2008 when positioning the surgical end-effector 2012 at a steep pitch angle relative to the patient 2014. In order to position the base 2010 of the surgical robot 2002 in the new position, the operator may, in some embodiments, disengage stabilizers of the surgical robot 2002 and manually push the base 2010 to the new position. In some embodiments, the display device 2016 may display guidance information to the operator to guide the operator's movement of the base 2010 in accordance with the determined motions. In some embodiments, repositioning the base 2010 may occur before positioning the robotic arm 2008. In this manner, the surgical robotic system 2000 can guide the operator to position the base 2010 such that the trajectories of the surgical robot 2008 provide increased range of the end-effector 2012 and/or increased freedom of movement of the robotic arm 2008 so as to avoid collisions with the patient 2014.

This example may be particularly useful, although not limited to, manually-controlled surgical robotic systems. In this manner, the surgeon and/or operator may be able to position an end-effector, such as the end-effector 2012, at steep angles and in close proximity to a patient without causing injury to the patient and/or damage to the surgical robot. By providing such protection, advantageous surgical movements may be achieved without the disadvantages associated with collisions of the robotic arm 2008.

In an autonomously-controlled surgical robotic system, it may be desirable to have components of the surgical robotic system perform similar operations as those described with respect to FIG. 22. As such, FIG. 23 is directed to performing remedial action(s) in a surgical robotic system, such as the surgical robotic system 2000 of FIG. 20, wherein the motor 2018 includes a first motor configured to translationally move the robotic arm 2008 based on a translational movement determined by the processor 2022, and a second motor configured to rotationally move the robotic arm 2008 and/or the end-effector 2012 based on the rotational movement determined by the processor 2022. In this regard, performing a remedial action can include, responsive to the proximity signal, determining a translational movement of the end-effector that will allow the end-effector to be moved toward a target location relative to the patient without collision of the robotic arm with the patient (block 2306 in FIG. 23). Performing such a remedial action may further include, responsive to the proximity signal, determining a rotational movement of the end-effector that will allow the end-effector to be further moved toward the target location relative to the patient without collision of the robotic arm with the patient (block 2308 in FIG. 23). Upon making such determinations, the processor may generate the translational commands for the at least one motor to translationally move the robotic arm based on the translational movement that is determined (block 2310 in FIG. 23), and generate the rotational commands for the at least one other motor to rotationally move the robotic arm based on the rotational movement that is determined (block 2312 in FIG. 23).

As discussed in the example above with respect to FIG. 22, in a surgery where the end-effector 2012 is being moved downward along the Y-axis toward the patient 2014, the processor 2022 may determine, based on the proximity signals captured by the sensor(s) 2030(A)-2030(C) and transmitted by the sensor processing computer 2006 to the processor 2022, that the robotic arm 2008 will soon collide with the patient 2014. In response to this determination, the processor 2022 may determine that the robotic arm 2008 should be moved in the positive Z-axis direction to avoid such a collision and that the end-effector 2012 should be rotated to be moved into a target location and disposition without collision. However, in contrast or in addition to the operations disclosed with respect to FIG. 22, the processor 2022 may choose to perform all or a part of these motions autonomously. In this regard, the processor 2022 can generate and transmit translational commands to the first motor of the motor 2018 configured to translationally move the robotic arm 2008.

Similarly, in sequence or simultaneously, the processor 2022 can generate and transmit rotational commands to the second motor of the motor 2018 configured to rotationally move the robotic arm 2008 or the end-effector 2012. In this manner, the determined movements of the end-effector 2012 and the robotic arm 2008 may be performed, at least in part, based on commands from the processor 2022. This example may be particularly useful, although not limited to, autonomously-controlled surgical robotic systems. In this manner, an end-effector, such as the end-effector 2012, may be positioned at steep angles and in close proximity to a patient without causing injury to the patient and/or damage to the surgical robot 2002 and without the need for excessive range of motion restrictions on the surgical robot 2002. In a similar manner to the benefits discussed above with regard to FIG. 22, providing such protection can allow for advantageous surgical movements without the disadvantages associated with collisions of the robotic arm 2008.

As noted above, FIG. 24 illustrates a flowchart 2400 directed to operations beyond the operations of determining when the robotic arm has collided with the patient or is predicted to collide with the patient based on the proximity signal, and performing a remedial action responsive to the determination. Specifically, the flowchart 2400 includes the operations of generating a data structure mapping distances between the robotic arm and the patient based on the proximity signal from the sensor (block 2406 in FIG. 24), and determining a pathway from the present location of the end-effector to the target location of the end-effector relative to the patient, wherein the determination of the pathway is constrained based on content of the data structure to avoid collision of the robotic arm with the patient (block 2408 in FIG. 24).

In one embodiment, the operations discussed with respect to FIG. 24 may be particularly useful in providing smooth surgical movements for a surgical robot during a surgery. For example, in a surgery where the end-effector 2012 is being moved along the X-axis, from the foot of the patient 2014 toward the head of the patient 2014, it may be beneficial to determine a pathway for the end-effector 2012 and the robotic arm 2008 so that the surgical robot 2002 does not collide with the patient 2014. In this regard, the processor 2022 may generate a data structure stored in the memory 2024 mapping distances between the robotic arm 2008 and the patient 2014 based on the proximity signals captured by the sensor(s) 2030(A)-2030(C). Based on the content of the data structure, the processor 2022 can determine a pathway from the present location of the end-effector 2012 (i.e., near the foot of the patient 2014) to the target location of the end-effector 2012 (i.e., near the head of the patient 2014) that is constrained to avoid a collision between the robotic arm 2008 and the patient 2014. For instance, the determined pathway may include a rise above the chest of the patient 2014 in the Y-axis direction so that the end-effector 2012 is able to avoid contact with the patient 2014 while traversing the path in the X-axis direction. Similarly, this approach may be applied to a variety of other combinations of pathways and directions. In this manner, the translational movement of the end-effector 2012 is determined based on the pathway that is determined.

Furthermore, like the embodiments discussed with respect to FIGS. 21-23, the processor 2022 may implement such operations by displaying guidance information to an operator or by generating and transmitting commands causing the motor 2018 to perform the determined movements. In this manner, an end-effector may be positioned at steep angles and in close proximity to a patient without causing injury to the patient and/or damage to the surgical robot and without the need for the surgical robot to slow down. In this regard, providing such protection and speed can allow for advantageous surgical movements designed to reduce tissue damage, blood loss, and scarring. Furthermore, the disadvantages associated with collisions of the robotic arm 2008 may also be avoided.

As indicated above, the sensor or sensors 2030(A)-2030(C) illustrated in FIG. 20 and referred to in FIGS. 21-24 may be organized in various configurations across different sensor locations and may be presented in various forms with various functions. Thus, the following paragraphs disclose different embodiments of the sensor or sensors 2030(A)-2030(C) as implemented in the embodiments discussed above.

In this regard, the sensor or sensors 2030(A)-2030(C) may include a pressure film configured to cause a proximity signal to be transmitted to the processor 2022 upon sensing pressure. In one embodiment, the pressure film is on the robotic arm 2008 in location 2030(A) such that the pressure film is connected to and extends along at least a portion of a surface of the robotic arm 2008. In this manner, the pressure film is connected to circuitry configured to output the proximity signal, such as the sensor processing computer 2006, indicating that a collision has occurred responsive to a force being exerted against the pressure film. In this example, the location of the sensor 2030(A) is on the lower portion 2008(B) of the robotic arm 2008. However, in additional embodiments, the location of the sensor 2030(A) may also be on any surface that may result in a collision of the robotic arm 2008, such as the upper portions 2008(A) of the robotic arm 2008, the hinging mechanism connecting the upper portion 2008(A) and the lower portion 2008(B), the base 2010, the end-effector 2012 or a portion thereof, the patient 2014, and/or any other feature of an operating environment.

In another embodiment, the sensor or sensors 2030(A)-2030(C) may be a load cell and/or a switch. For example, the load cell and/or the switch may be located on the robotic arm 2008 such that the load cell and/or switch is connected to the robotic arm 2008. In this manner, the load cell and/or switch is connected to circuitry configured to output the proximity signal, such as the sensor processing computer 2006, indicating that a collision has occurred responsive to a force being exerted against the load cell and/or switch. In a similar manner to the pressure film, the load cell and/or switch may be positioned at a variety of relevant locations.

In another embodiment, the sensor or sensors 2030(A)-2030(C) may be a light source and a light sensor. For example, a light source affixed to the robotic arm 2008 at the position 2030(A) may typically emit a beam of light that is received by a light sensor affixed to the robotic arm 2008 at the position 2030(A) as well. By having the light sensor and the light source spaced apart along the robotic arm 2008, the light sensor can be configured to receive light from the light source when a light conductive pathway between the light source and the light sensor is not blocked. However, when the pathway is blocked, e.g., by an object that is about to collide with the robotic arm, the light sensor may provide a proximity signal to the sensor processor computer 2006. In a similar manner to the pressure film and the load cell and/or switch discussed above, the light source and light sensor may be positioned at a variety of relevant locations in a surgical environment.

In another embodiment, the sensor or sensors 2030(A)-2030(C) may be an electro-conductive pad system. For example, an electroconductive pad system including a first electroconductive pad and a second electroconductive pad may be provided in the surgical robotic system 2000 illustrated in FIG. 20. Specifically, a first electroconductive pad may be located at position 2030(A) and a second electroconductive pad may be located at position 2030(B) on the patient 2014. In this manner, the first electroconductive pad is connected to and extends along at least a portion of a surface of the robotic arm 2008 and the second electroconductive pad is connected to and extends along at least a portion of the patient 2014. By configuring the electroconductive pad system to generate a current when the first and second electroconductive pads comes into contact, the electroconductive pad system can cause circuitry, such as the sensor processor computer 2006, to provide a proximity signal to the processor 2022 indicating that a collision has occurred. In this regard, the electroconductive pad system is able to send a proximity signal to the processor 2022 for determining whether a collision has occurred between the robotic arm 2008 and the patient 2014. In a similar manner to embodiments discussed above, the electroconductive pad system may be positioned at a variety of relevant locations in a surgical environment.

In another embodiment, the sensor or sensors 2030(A)-2030(C) may be a distance ranging circuit configured to output a proximity signal providing an indication of distance between a portion of the robotic arm 2008 and the patient 2014. In this regard, in one embodiment, the distance ranging circuit may be connected to the robotic arm 2008 at position 2030(A) and configured to determine distance in a direction away from the robotic arm 2008. In one embodiment, determining the distance in the distance ranging circuit can include using a time-of-flight measurement system. In such a system, an emitter emits a pulse of a first type of energy and a detector receives a reflection of the pulse from an incident area of the patient 2014. Upon receipt, a processor determines the distance the pulse traveled based on the travel time of the pulse between emission and receipt. In at least one example, the emitter may be located at position 2030(C) and the detector may be located at another position in the surgical environment, such as 2030(A), 2030(B), or even 2030(C). In some embodiments, the processor may be a processor located in the sensor processing computer 2006, the processor 2022 in the surgical control computer 2004, or a processor located elsewhere in the surgical robotic system 2000. Upon determining the distance, the processor of the time-of-flight measurement system generates a proximity signal based on the distance that is determined. In at least one embodiment, the first type of energy is sonic energy or electromagnetic energy (e.g., RF signal). In at least an additional embodiment, the sonic energy comprises ultrasonic frequency signals and the electromagnetic energy comprises one of radio frequency signals, microwave frequency signals, infrared frequency signals, visible frequency signals, and ultraviolet frequency signals. In yet another embodiment, the emitter and the detector of the time-of-flight measurement system are connected to the robotic arm 2008.

In other embodiments, the sensor or sensors 2030(A)-2030(C) may be a probe configured to measure at least one location on a patient, such as the patient 2014. The probe may be tracked with, or registered to, the same tracking method that tracks the location of the robotic arm 2008. In this regard, the operations by the surgical control computer 2004 can include determining a pathway from the present location of the end-effector 2012 to the target location of the end-effector 2012 relative to the patient 2014, and determining whether the pathway would result in the robotic arm 2008 colliding with the patient 2014 based on the at least one location on the patient 2014. In response to determining that the pathway would result in the robotic arm 2008 colliding with the patient 2014, a proximity signal can be generated indicating that the robotic arm 2008 is predicted to collide with the patient 2014.

In other embodiments, the sensor or sensors 2030(A)-2030(C) may be a camera system configured to determine distances between a robotic arm, such as the robotic arm 2008, and an array of tracking markers on a patient, such as patient 2014. In this manner, the camera system can generate a proximity signal based on the distances that are determined.

With regard to the surgical control computer 2004, the processor 2022 may include one or more data processing circuits, such as a general purpose and/or special purpose processor (e.g., microprocessor and/or digital signal processor), which may be collocated or distributed across one or more data networks. The processor 2022 is configured to execute computer program instructions among program code 2026 in the memory 2024, described below as a computer readable medium, to perform some or all of the operations and methods for one or more of the embodiments disclosed herein for a surgical control computer 2004. The network interface circuit 2028 is configured to communicate with another electronic device, such as a server(s) and/or the surgical robot 2002, through a wired network (e.g., ethernet, USB, etc.) and/or wireless network (e.g., Wi-Fi, Bluetooth, cellular, etc.).

It is contemplated that the surgical robotic systems disclosed herein are configured for use in any type of surgical procedures, including but not limited to, surgeries in trauma or other orthopedic applications (such as the placement of intramedullary nails, plates, and the like), cranial, neuro, cardiothoracic, vascular, colorectal, oncological, dental, and other surgical operations and procedures.

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belong. It will be further understood that terms, such as those defined in commonly used dictionaries, should be interpreted as having a meaning that is consistent with their meaning in the context of this specification and the relevant art and will not be interpreted in an idealized or overly formal sense unless expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a non-transitory computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Although several embodiments of inventive concepts have been disclosed in the foregoing specification, it is understood that many modifications and other embodiments of inventive concepts will come to mind to which inventive concepts pertain, having the benefit of teachings presented in the foregoing description and associated drawings. It is thus understood that inventive concepts are not limited to the specific embodiments disclosed hereinabove, and that many modifications and other embodiments are intended to be included within the scope of the appended claims. It is further envisioned that features from one embodiment may be combined or used with the features from a different embodiment(s) described herein. Moreover, although specific terms are employed herein, as well as in the claims which follow, they are used only in a generic and descriptive sense, and not for the purposes of limiting the described inventive concepts, nor the claims which follow. The entire disclosure of each patent and patent publication cited herein is incorporated by reference herein in its entirety, as if each such patent or publication were individually incorporated by reference herein. Various features and/or potential advantages of inventive concepts are set forth in the following claims.

The invention could be defined inter alia by the following examples:
1. A surgical robotic system comprising: a surgical robot comprising a robotic arm and a controller, wherein the robotic arm is configured to be connectable to a surgical end-effector and configured to position the surgical end-effector relative to a patient; a sensor configured to output a proximity signal indicating, while the robotic arm is positioned adjacent to a patient, proximity of the robotic arm to the patient; and a surgical control computer comprising: at least one processor connected to receive the proximity signal from the sensor; and at least one memory storing program instructions executed by the at least one processor to perform operations comprising: determining when the robotic arm has collided with the patient or is predicted to collide with the patient based on the proximity signal; and performing a remedial action responsive to the determination.
2. The surgical robotic system of Example 1, wherein the operations for performing the remedial action responsive to the determination, comprise: controlling a display device to display a collision warning to an operator and/or controlling an audio generation device to output an audible collision warning to the operator.
3. The surgical robotic system of Example 1, further comprising a motor connected to move the robotic arm responsive to commands, wherein the operations for performing the remedial action responsive to the determination, comprise: controlling the motor via a command to inhibit movement of the robotic arm in a direction toward where the robotic arm has collided with the patient or is predicted to collide with the patient.
4. The surgical robotic system of Example 1, wherein the operations for performing the remedial action responsive to the determination, comprise: responsive to the proximity signal, determining a translational movement of the end-effector that will allow the end-effector to be moved from a present location toward a target location relative to the patient without collision of the robotic arm with the patient; and displaying guidance information to an operator that guides the operator's movement of the end-effector based on the translational movement that is determined.
5. The surgical robotic system of Example 4, wherein the operations for performing the remedial action responsive to the determination, further comprise: responsive to the proximity signal, determining a rotational movement of the end-effector that will allow the end-effector to be further moved toward the target location relative to the patient without collision of the robotic arm with the patient, wherein the guidance information displayed to the operator guides the operator's movement of the end-effector based on the translational movement and the rotational movement that is determined.
6. The surgical robotic system of Example 4, wherein the operations by the surgical control computer further comprise: generating a data structure mapping distances between the robotic arm and the patient based on the proximity signal from the sensor; and determining a pathway from the present location of the end-effector to the target location of the end-effector relative to the patient, wherein the determination of the pathway is constrained based on content of the data structure to avoid collision of the robotic arm with the patient, wherein the translational movement of the end-effector is determined based on the pathway that is determined.
7. The surgical robotic system of Example 1, further comprising: at least one motor connected to translationally move the robotic arm responsive to translational commands, wherein the operations for performing the remedial action responsive to the determination, comprise: responsive to the proximity signal, determining a translational movement of the end-effector that will allow the end-effector to be moved toward a target location relative to the patient without collision of the robotic arm with the patient; and generating the translational commands for the at least one motor to translationally move the robotic arm based on the translational movement that is determined.
8. The surgical robotic system of Example 7, further comprising: at least one other motor connected to rotationally move the robotic arm responsive to rotational commands, wherein the operations for performing the remedial action responsive to the determination, further comprise: responsive to the proximity signal, determining a rotational movement of the end-effector that will allow the end-effector to be further moved toward the target location relative to the patient without collision of the robotic arm with the patient; and generating the rotational commands for the at least one other motor to rotationally move the robotic arm based on the rotational movement that is determined.
9. The surgical robotic system of Example 7, wherein the operations by the surgical control computer further comprise: generating a data structure mapping distances between the robotic arm and the patient based on the proximity signal from the sensor; and determining a pathway from the present location of the end-effector to the target location of the end-effector relative to the patient, wherein the determination of the pathway is constrained based on content of the data structure to avoid collision of the robotic arm with the patient wherein the translational commands are generated for the at least one motor to translationally move the robotic arm based on the pathway that is determined.
10. The surgical robotic system of Example 1, wherein: the sensor comprises a pressure film connected to and extending along at least a portion of a surface of the robotic arm, wherein the pressure film is connected to circuitry configured to output the proximity signal indicating that a collision has occurred responsive to a force being exerted against the pressure film.
11. The surgical robotic system of Example 1, wherein: the sensor comprises at least one of a load cell connected to the robotic arm and a switch connected to the robotic arm, and is connected to circuitry configured to output the proximity signal indicating that a collision has occurred responsive to a force being exerted against the at least one of the load cell connected to the robotic arm and the switch.
12. The surgical robotic system of Example 1, wherein: the sensor comprises a light sensor and a light source spaced apart along the robotic arm, wherein the light sensor is configured to receive light from the light source when a light conductive pathway between the light source and the light sensor is not blocked, and to provide the proximity signal responsive to the pathway being blocked.
13. The surgical robotic system of Example 1, wherein: the sensor comprises an electroconductive pad system comprising a first electroconductive pad and a second electroconductive pad, wherein the electroconductive pad system is configured to generate a current when one of the first and second electroconductive pads, connected to and extending along at least a portion of a surface of the robotic arm, is coupled to another of the first and second electroconductive pads, connected to and extending along at least a portion of the patient, wherein the electroconductive pad system is connected to circuitry configured to provide the proximity signal indicating that a collision has occurred responsive to the current being generated by the electroconductive pad system.
14. The surgical robotic system of Example 1, wherein: the sensor comprises a distance ranging circuit that outputs the proximity signal providing an indication of distance between a portion of the robotic arm and the patient.
15. The surgical robotic system of Example 14, wherein: the distance ranging circuit is connected to the robotic arm and configured to determine distance in a direction away from the robotic arm.
16. The surgical robotic system of Example 14, wherein the distance ranging circuit comprises a time-of-flight measurement system comprising: an emitter configured to emit a pulse of a first type of energy; a detector configured to receive the pulse; and a processor configured to determine the distance the pulse traveled based on the travel time of the pulse between emission and receipt, and generate the proximity signal based on the distance that is determined, wherein the first type of energy is sonic energy or electromagnetic energy.
17. The surgical robotic system of Example 16, wherein: sonic energy comprises ultrasonic frequency signals; and electromagnetic energy comprises one of radio frequency signals, microwave frequency signals, infrared frequency signals, visible frequency signals, and ultraviolet frequency signals.
18. The surgical robotic system of Example 16, wherein: the emitter and the detector are connected to the robotic arm.
19. The surgical robotic system of Example 1, wherein: the sensor comprises a probe configured to measure at least one location on the patient, wherein the operations by the surgical control computer further comprise: determining a pathway from the present location of the end-effector to the target location of the end-effector relative to the patient; determining whether the pathway would result in the robotic arm colliding with the patient based on the at least one location on the patient; and in response to determining that the pathway would result in the robotic arm colliding with the patient, generating a proximity signal indicating that the robotic arm is predicted to collide with the patient.
20. The surgical robotic system of Example 1, wherein: the sensor comprises a camera system configured to determine distances between the robotic arm and an array of tracking markers on the patient, and to generate the proximity signal based on the distances that are determined.

## Claims

1. A surgical robotic system comprising:
- a surgical robot comprising a robotic arm and a controller, wherein the robotic arm is configured to be connectable to a surgical end-effector and configured to position the surgical end-effector relative to a patient;
- a sensor configured to output a proximity signal indicating, while the robotic arm is positioned adjacent to a patient, proximity of the robotic arm to the patient; and
- a surgical control computer comprising:
- at least one processor connected to receive the proximity signal from the sensor; and
- at least one memory storing program instructions executed by the at least one processor to perform operations comprising:
- determining when the robotic arm has collided with the patient or is predicted to collide with the patient based on the proximity signal; and
- performing a remedial action responsive to the determination.

2. The surgical robotic system of Claim 1, wherein the operations for performing the remedial action responsive to the determination, comprise: controlling a display device to display a collision warning to an operator and/or controlling an audio generation device to output an audible collision warning to the operator.

3. The surgical robotic system of Claim 1, further comprising:
- a motor connected to move the robotic arm responsive to commands,
- wherein the operations for performing the remedial action responsive to the determination, comprise:
- controlling the motor via a command to inhibit movement of the robotic arm in a direction toward where the robotic arm has collided with the patient or is predicted to collide with the patient.

4. The surgical robotic system of Claim 1, wherein the operations for performing the remedial action responsive to the determination, comprise:
- responsive to the proximity signal, determining a translational movement of the end-effector that will allow the end-effector to be moved from a present location toward a target location relative to the patient without collision of the robotic arm with the patient; and
- displaying guidance information to an operator that guides the operator's movement of the end-effector based on the translational movement that is determined.

5. The surgical robotic system of Claim 4, wherein the operations for performing the remedial action responsive to the determination, further comprise:
- responsive to the proximity signal, determining a rotational movement of the end-effector that will allow the end-effector to be further moved toward the target location relative to the patient without collision of the robotic arm with the patient,
- wherein the guidance information displayed to the operator guides the operator's movement of the end-effector based on the translational movement and the rotational movement that is determined.

6. The surgical robotic system of Claim 4, wherein the operations by the surgical control computer further comprise:
- generating a data structure mapping distances between the robotic arm and the patient based on the proximity signal from the sensor; and
- determining a pathway from the present location of the end-effector to the target location of the end-effector relative to the patient, wherein the determination of the pathway is constrained based on content of the data structure to avoid collision of the robotic arm with the patient,
- wherein the translational movement of the end-effector is determined based on the pathway that is determined.

7. The surgical robotic system of Claim 1, further comprising:
- at least one motor connected to translationally move the robotic arm responsive to translational commands,
- wherein the operations for performing the remedial action responsive to the determination, comprise:
- responsive to the proximity signal, determining a translational movement of the end-effector that will allow the end-effector to be moved toward a target location relative to the patient without collision of the robotic arm with the patient; and
- generating the translational commands for the at least one motor to translationally move the robotic arm based on the translational movement that is determined.

8. The surgical robotic system of Claim 7, further comprising:
- at least one other motor connected to rotationally move the robotic arm responsive to rotational commands,
- wherein the operations for performing the remedial action responsive to the determination, further comprise:
- responsive to the proximity signal, determining a rotational movement of the end-effector that will allow the end-effector to be further moved toward the target location relative to the patient without collision of the robotic arm with the patient; and
- generating the rotational commands for the at least one other motor to rotationally move the robotic arm based on the rotational movement that is determined.

9. The surgical robotic system of Claim 7, wherein the operations by the surgical control computer further comprise:
- generating a data structure mapping distances between the robotic arm and the patient based on the proximity signal from the sensor; and
- determining a pathway from the present location of the end-effector to the target location of the end-effector relative to the patient, wherein the determination of the pathway is constrained based on content of the data structure to avoid collision of the robotic arm with the patient,
- wherein the translational commands are generated for the at least one motor to translationally move the robotic arm based on the pathway that is determined.

10. The surgical robotic system of Claim 1, wherein:
- the sensor comprises a pressure film connected to and extending along at least a portion of a surface of the robotic arm, wherein the pressure film is connected to circuitry configured to output the proximity signal indicating that a collision has occurred responsive to a force being exerted against the pressure film.

11. The surgical robotic system of Claim 1, wherein:
- the sensor comprises at least one of a load cell connected to the robotic arm and a switch connected to the robotic arm, and is connected to circuitry configured to output the proximity signal indicating that a collision has occurred responsive to a force being exerted against the at least one of the load cell connected to the robotic arm and the switch.

12. The surgical robotic system of Claim 1, wherein:
- the sensor comprises a light sensor and a light source spaced apart along the robotic arm, wherein the light sensor is configured to receive light from the light source when a light conductive pathway between the light source and the light sensor is not blocked, and to provide the proximity signal responsive to the pathway being blocked.

13. The surgical robotic system of Claim 1, wherein:
- the sensor comprises an electroconductive pad system comprising a first electroconductive pad and a second electroconductive pad, wherein the electroconductive pad system is configured to generate a current when one of the first and second electroconductive pads, connected to and extending along at least a portion of a surface of the robotic arm, is coupled to another of the first and second electroconductive pads, connected to and extending along at least a portion of the patient,
- wherein the electroconductive pad system is connected to circuitry configured to provide the proximity signal indicating that a collision has occurred responsive to the current being generated by the electroconductive pad system.

14. The surgical robotic system of Claim 1, wherein: the sensor comprises a distance ranging circuit that outputs the proximity signal providing an indication of distance between a portion of the robotic arm and the patient.

15. The surgical robotic system of Claim 14, wherein: the distance ranging circuit is connected to the robotic arm and configured to determine distance in a direction away from the robotic arm.
